# EUROPEAN PATENT APPLICATION

(11) **EP 1 477 567 A1**
(43) Date of publication of application: **17.11.2004**
(21) Application number: 03010844.3
(22) Date of filing: 14.05.2003
(51) Int. Cl.: C12P 21/02, C12N 13/00, C12N 15/861

(54) **Method for recombinant production of mammalian proteins and proteins obtainable by such method**

(71) Applicant: Axxima Pharmaceuticals AG, 81377 München (DE)
(72) Inventor: Cotten, Matthew, 80799 Munich (DE)

(57) **Abstract**

The present invention relates to a recombinant expression system using adenoviruses for the production of mammalian proteins, especially enzymatic proteins, and a method for inactivating the viruses by means of treatment with intercalating agents plus irradiation without substantially affecting the biological activity of the proteins.

## Description

The present invention relates to a method for the recombinant production of mammalian proteins as well as proteins obtainable by such a method.

### Background of the Invention

Expression of protein enzyme molecules in sufficient quantities for biochemical and pharmaceutical studies can be difficult with many of the standard protein expression systems. Bacterial systems for example generate large amounts of recombinant protein. However, often the protein is improperly folded and can be insoluble. Furthermore, modification by phosphorylation or appropriate glycosylation, which may be essential for full activity, does not occur in bacterial systems. Baculovirus or yeast expression systems are a possible alternative to provide eukaryotic expression and post-translational modification. However, the establishment of these systems can be time consuming and ultimately, the protein is produced in a non-mammalian cell type without full mammalian post-translation folding and modification. For the generation of mammalian proteins where native activity, solubility and post-translational modification is crucial, it would be of great utility to have a reliable system for generating useful quantities of these molecules.

Recombinant adenoviruses have been used for a number of years as vectors to deliver genetic material to cells or organisms and for the production of recombinant proteins (reviewed in W. C. Russell, Update on adenovirus and its vectors; J. Gen. Virol. 2000, 81, 2573 to 2604). If appropriate measures are taken to ensure transcription and translation of the transgene and transgene product during the late stages of virus growth, the recombinant protein can represent a significant proportion of the infected cell lysate. This is due to several factors including the virus shut down of host protein synthesis and the preferential splicing and nuclear export of viral messages late in infection (reviewed in K. L. Berkner, Expression of heterologous sequences in adenoviral vectors; Curr. Top. Microbiol. Immunol. 1992, 158, 39-66). One major complication associated with recombinant adenovirus use for protein production has been the residual adenovirus present in the protein preparations. Because adenovirus titers after productive infection are typically in the range of 10⁹ to 10¹² virions/ml and the non-enveloped virion is stable to detergents, high salt, and sonication, significant amounts of active virus can be found in preparations of the recombinant protein.

### Objects and Summary of the Invention

Thus, it is object of the present invention to provide a method for expressing mammalian proteins, inactivating the adenoviruses used as expression system without substantially affecting said mammalian proteins, and purifying the overexpressed mammalian proteins.

The object of the present invention is solved by the teaching of independent claims 1 and 12. Further advantageous features, aspects and details of the invention are evident from the dependent claims, the description, the examples, and the figures of the present application.

The use of adenovirus as a protein expression system is not a recent development. Recombinant forms of adenovirus were some of the first viruses used to express proteins in eukaryotic cells. Unfortunately, the initial experiments were performed in cells that have limited amounts of the adenovirus receptor CAR and the expression levels were not very strong. In addition, early methods of recombinant adenovirus construction were complicated and lengthy, and finally, although the replication defective adenoviruses pose no health risk, many regulatory agencies require that the viruses and any material containing residual virus (e.g purified proteins derived from the viruses) must be handled with appropriate containment. Elimination of adenovirus is difficult, unlike enveloped virus, the non-enveloped adenovirus is stable to detergents, chloroform and most conditions used to purify native proteins. Thus, there has been only a modest use of recombinant adenovirus systems as a source for recombinant protein, and adenovirus use in this field has been largely overshadowed by bacterial and baculovirus systems.

With the vast quantity of genome sequence data now available, it is clear that new methods of generating and characterizing the products of genes are required. In recent years, adenovirus vectors have been found to be an extremely versatile alternative to transient transfection or stable cell line production to drive novel gene expression, without impairing normal cellular functions. An added advantage is the ability to use the same vectors in vivo for experiments in animal models. A variety of simplified adenovirus construction techniques have now been developed that accelerate the construction of generated clonal viruses.

It is demonstrated here that with appropriate optimization of infection conditions, standard, first generation E1-deficient recombinant adenovirus vectors containing CMV immediate early enhancer/promoter driven transgenes can serve as a useful source of recombinant proteins, particularly kinases and phosphatases. An affinity tag can be included on the recombinant protein to facilitate purification. The optimized system can provide 0.5 to 2 mg of pure, active protein starting from 18x185 cm² flasks of 293 cells. It could be demonstrated that the system can successfully generate active eukaryotic kinase molecules that could not be produced by either bacterial or baculovirus systems in an active form. A method of demonstrating purity of a kinase preparation was developed using genetically inactive kinase mutant purified to the same extent. This method shows that for most kinases, the purified material is free of contaminating host enzymes, although one important exception is described. The question of contaminating adenovirus was addressed. It could be shown that even with affinity purification a detectable quantity of active adenovirus is present in the final enzyme preparation. Most importantly, according to a specific aspect of the present invention a method of treatment of a recombinant protein preparation with 8-methoxy psoralen combined with exposure to long wave UV light was= identified that completely eliminates residual replicating adenovirus in the recombinant protein preparation. This treatment is gentle to proteins and no substantial decline in recombinant enzyme activity occurs with this method.

Thus, the present invention describes the imaginative combination of mammalian cells which are infected by adenoviruses used as expression system in order to produce mammalian proteins. Said combination is able to overcome the above-mentioned drawbacks of the prior art using bacterial or baculovirus expression systems.

Thus, independent claim 1 discloses a method for recombinant production of mammalian proteins comprising the following steps:
a) providing a recombinant adenovirus as expression system;
b) infecting mammalian cells with the recombinant adenovirus expresion sytem to produce an active mammalian protein;
c) inactivating the adenovirus in the preparation by use of an UV-sensitive intercalating agent and UV irradiation; and
d) separation and/or purification of the produced mammalian protein.

Moreover, the present invention relates to a mammalian protein obtainable by the above-mentioned method.

### Detailed Description of the Preferred Embodiments

According to an aspect of the present invention, in step b) the active mammalian protein is produced by inclusion of at least one of the host-mediated step(s) during protein production to ensure either approriate folding or post-translational modification for full activity of the recombinant protein.

Adenovirus expression systems are flexible with regard to the cells used for production of biologic material. Baculovirus systems, based on an insect virus, are limited to insect cells for protein production. Because many applications, especially medical drug research, seek compounds that interact with mammalian and especially human proteins, the production of target proteins in human or mammalian systems is essential. Thus the proper folding, interaction with chaperones and post-translational modification (e.g. glycosylation, acetylation, phosphorylation) can be highly influenced by the cell type. Activation of many kinases requires modification by other kinases, and if the host kinases either do not recognize the mammalian protein (in baculovirus) or do not exist (E. coli), the target enzyme must by activated by other methods. However, for certain kinases, the adenovirus systems itself activates the pathway and the kinases are thus produced in an active form (see below for p38). The baculovirus system has been shown to work poorly to produce active mammlian raf-1 unless the mammalian chaperone cdc37 is provided to the insect cells (Grammatikakis et al., 1999). Cdk9cyclin T1 maturation also requires cdc37 (O'Keeffe et al., 2000) and this limitation is not seen when cdk9/cyclin T1 is produced in mammalian, especially human cells.

Preferably, the inventive method is used to produce human proteins and most preferably human kinases and human phosphatases. Thus, it is advantageous that human cells can be used for this method in order to insure, for instance, proper folding of the produced proteins, such as kinases and phosphatases. In the case that human cells are used, extra measures to generate post-translational translational modifications, such as ac(et)ylation, phosphorylation, glycosylation, ubiquitination, sumo modification etc. and/or interactions with chaperones, are not required. The use of human cells has not been pursued in the past, because such fast, efficient methods like the adenovirus, was were not readily available. Furthermore, the fear of contaminating mammalian enzymatic activity (e.g. kinases) was abundant.

Furthermore, protein production using a combination of recombinant adenovirus expression with an affinity purification method is described. Advantages of this system are the use of human cells which ensures appropriate post-translational modification of mammalian proteins and the ability to use the same recombinant adenovirus in gene expression experiments as well as for protein production. The optimum infection and growth conditions were identified to give production of a recombinant protein kinase at the same level as the highly expressed viral capsid proteins. An affinity tag for rapid purification of the transgene products is shown to function well in combination with an adenovirus system. Finally, one major concern with the use of recombinant adenovirus is the presence of residual virus in the purified recombinant protein. It is demonstrated here, that even affinity-purified protein preparations contain detectable adenovirus. However, the simple treatment of the material with a UV-sensitive intercalating agent, such as 8-methoxy psoralen, combined with long wavelength UV light destroys any residual adenovirus without damaging protein function, such as kinase function.

Inactivation of the adenoviruses is achieved by means of an intercalating agent and UV irradiation. One of the most common intercalating agents is psoralen and its derivatives (Hanson CV., Photochemical inactivation of viruses with psoralens: an overview. Blood Cells. 1992; 18(1): 7-25).

Also other methods of selectively inactivating the contaminating adenovirus can be considered. The important requirements which have to be fulfilled by said inactivation methods are:
1. the ability to irreversibly block an essential component of adenovirus replication and 2. no impairment of the enzymatic activity of the target protein.

In US-A-4 545 987, for instance, a method for producing a vaccine for inoculation of a mammalian host susceptible to infection by blue-tongue virus (BTV) is described. Said method comprises inactivation of at least one BTV serotype by exposure to long wavelength ultraviolet light in the presence of a furocoumarin as intercalating agent. The irradiation is carried out until a non-infectious degree of BTV is present in the sample used to prepare the vaccine. Thus, the vaccine contains non-infectious virus particles in order to immunize the treated individual against infectious of BTV. It is not mentioned whether any biologically active components beside said BT viruses retain their biological activity.

EP-A-0 184 331 describes a method for rapid inactivation of Human Tumor Leukemia Virus III (HTLV III) wherein new psoralen derivatives are used. Said method is capable of rapid inactivation of HTLV III without substantial loss of antigenicity. The HTLV III is inactivated by exposing a sample containing the HTLV III and a psoralen derivative to electromagnetic radiation (light) having a wavelength of 320 to 380 nm. It is stated that the nucleocapsid, capsid, and viral envelope of HTLV III remains substantially unaltered, but the effect of irradiation on any other biologically active component contained in said sample is not examined and discussed.

WO 95/18530 describes a general method for inactivating a virus present in a blood sample by means of a compound, such as psoralen or hypericin, which has a nucleic acid affinity and can also be activated to an excited state in which said compound covalently binds to a nucleic acid in such a way as to effectively disable said nucleic acid. The effect of said method on other components contained in the blood was not measured and discussed.

US-A-6 136 586 discloses methods of inactivating viruses and other organism in cell-containing or biopolymer-containing compositions without significant alteration of proteins or other biopolymers by contacting such compositions with selective ethyleneimine oligomers inactivating agents. Said methods do not incorporate any use of UV light in order to obtain inactivated viruses.

In carrying out the present invention, the UV-sensitive intercalating agent is dissolved in a solvent which is substantially inert and sufficiently polar to allow for dissolution of the UV-sensitive intercalating agent. Preferably water-based solutions are used. Said solution of the UV-sensitive intercalating agent is combined with the viral suspension to obtain the sample which comprises biologically active components, at least one UV-sensitive intercalating agent, and the viruses. Preferably, psoralen is dissolved in DMSO and then diluted into water.

The use of dioxetanes, psoralen, furocoumarin, hypericin, and derivatives thereof, such as 5-methoxypsoralen, 8-methoxypsoralen (8-MOP), 4,5',8-trimethylpsoralen (TMP), 4'-hydroxymethyl-4,5',8-trimethylpsoralen (HMT), 4'-aminomethyl-4,5',8-trimethylpsoralen (AMT), 4-methylpsoralen, 4,4'-dimethylpsoralen, 4,5'-dimethylpsoralen, 4',8-dimethylpsoralen, and 4'-methoxymethyl-4,5',8-trimethylpsoralen (MMT) for virus inactivation is well known in both, the patent and non-patent literature. EP-A-0 184 331 describes in detail the conditions for the use of psoralen and various derivatives thereof for the inactivation of different kinds of viruses. Preferred is the use of 8-MOP and AMT.

Said psoralen derivatives preferably bear substituents such as methyl and/or alkoxy groups particularly on 1 to 3 carbon atoms. Furthermore, hydroxy, alkoxy, hydroxymethyl, aminoalkyl, alkyl aminoalkyl, and dialkyl aminoalkyl are also common substituents preferably on 1 to 6 carbon atoms, more usually on 2 to 4 carbon atoms.

Isaacs et al, Biochemistry 1977, 16, 1058-1064, describe the synthesis of several psoralen derivatives and their photoreactivity with double-stranded RNA. Hearst and Thiry, Nucleic Acid Research 1977, 4, 1339-1347; and Hanson et al., J. Gen. Virol. 1978, 40, 345-358, describe the photochemistry of various psoralen derivatives with animal viruses.

Because of the relatively small size of the intercalating agent, said intercalating agent is readily taken up by the virus by diffusing through the viral capsid. Once inside the virus, the intercalating agent intercalates between the base pairs of the viral nucleic acids. After addition of the intercalating agent to the sample, said sample is irradiated with UV light in order to cause the intercalating agent to be raised to an excited state. After having reached said excited state, the intercalating agent binds covalently to the viral nucleic acid, thereby impairing the operability of the bound nucleic acids.

The intercalating agents may be used individually or in combination. Each intercalating agent may be present in the sample in amounts ranging from about 0.01 µg/ml to 1 mg/ml, preferably from about 0.5 µg/ml to 0.5 mg/ml, more preferably from 0.01 mg - 0.3 mg, and most preferably from 0.05 mg to 0.2 mg.

The intercalating agent may be added to the sample in a single addition or in multiple additions, where the virus is irradiated between additions. Usually the number of additions will be from 1 to 5, more usually from 1 to 4, preferably from 2 to 4, and most preferably from 2 to 3.

The total amount of intercalating agents added to the sample will be sufficient to provide a concentration of at least about 0.005 mg/ml to about 1 mg/ml, usually not more than about 0.8 mg/ml, and preferably not more than about 0.5 mg/ml. Since a substantial proportion of the intercalating agent will have reacted with the nucleic acid between addition, the total concentration of intercalating agent in the sample will generally not exceed 0.1 mg/ml.

As used herein, the term "inactivation" refers to a modification of a virus in order to eliminate its infectivity and/or replication capacity.

The irradiation is carried out in presence of a sufficient amount of at least one of the above-mentioned UV-sensitive intercalating agents to provide for substantially complete inactivation of the virus by the use of long wavelength (300 - 400 nm) ultraviolet light (UVA). Preferably, ultraviolet light of a wavelength in the range of 320 - 380 nm, more preferably in the range of 350 - 375 nm, still more preferably in the range of 360 - 370 nm, and most preferably in the range of 365 - 366 nm is used.

The ultraviolet light which is employed has usually a wavelength in the range from 300 - 400 nm and the intensity thereof ranges from about 0.1 mW/cm² to about 200 mW/cm², preferably from about 1.0 mW/cm² to about 80 mW/cm², more preferably from about 4.0 mW/cm² to about 40 mW/cm², still more preferably from about 8.0 mW/cm² to about 20 mW/cm², most preferably from about 10.0 mW/cm² to about 15 mW/cm² at 3 cm.

As light sources conventional UV lamps or lasers, such as XeCl eximer laser (wavelength: >305 nm), and Nd:YAG laser, may be used.

Optionally, the irradiation can be carried out under an inert atmosphere such as argon, neon, carbon dioxide, helium or nitrogen.

The total time for the irradiation varies depending upon the light intensity, the typand/or concentration of the intercalating agent(s), the concentration of viruses, and the kind of medium used, wherein the intensity of the irradiation may vary in the medium. Usually, sample volumes of less than 1 ml are irradiated for 30 to 45 minutes using the UV-lamp listed above.

The time between additions of the intercalating agent(s), where the intercalating agent(s) is/are added incrementally, will vary from about 1 to 24 hours, preferably from 2 to 20 hours, and most preferably from 4 to 10 hours.

The temperature for the irradiation is usually under 65°C and over -20°C. Preferably, the temperature for irradiation is in the range of -10°C to 55°C, more preferably in the range from -10°C to 40°C, still more preferably in the range from, -5°C to 30°C, and most preferably in the range from -5°C to 10°C.

The sample can be continuously irradiated or irradiated in alternating periods of irradiation and non-irradiation. During irradiation the sample may be maintained still, stirred, or circulated. The circulation can be performed in a closed loop system or in a single pass system ensuring that all of the samples have been subjected to irradiation. Using a continuous irradiation process, pump rates larger than 0.5 ml/s, preferably 1.0 ml/s, more preferably 5 ml/s, and most preferably larger than 10 ml/s can be achieved.

Optionally, a small amount of a singlet oxygen scavenger and/or an antioxidant may be added to the sample during the virus inactivation. Suitable singlet oxygen scavengers and antioxidants can be selected from the group comprising vitamin E (DL-a-tocopherol), vitamin C (L-ascorbic acid), co-enzyme Q10, zinc, selenium, N-acetyl-L-cycteine, N-acetyl-S-farnesyl-L-cysteine, Bilirubin, caffeic acid, citric acid, CAPE, catechin, ceruloplasmin, Coelenterazine, copper diisopropylsalicylate, deferoxamine mesylate, R-(-)-deprenyl, DMNQ, DTPA dianhydride, Ebselen, ellagic acid, (-)-epigallocatechin, L-ergothioneine, EUK-8, Ferritin, glutathione, glutathione monoethylester, a-lipoic acid, Luteolin, Manoalide, MCI-186, MnTBAP, MnTMPyP, morin hydrate, NCO-700, NDGA, p-Nitroblue, propyl gallate, Resveratrol, rutin, silymarin, L-stepholidine, taxifolin, tetrandrine, tocopherol acetate, tocotrienol, Trolox® , U-74389G, U-83836E, and uric acid (all available from Calbiochem, San Diego, CA, U.S.A.), and phenolic compounds such as BHA (butylated hydroxyanisole), BHT (butylated hydroxytoluene), propyl gallate, TBHQ (tert-butyl hydroquinone), tocopherols, lecithin, gums and resin guiac, THBP (trihydroxybutyrophenone), dithioerythritol, sodium thionite, thiodipropionic acid, dilauryl thiodipropionate, and glycines.

The amount of singlet oxygen scavenger and/or antioxidants will generally for each compound be at a concentration of about 0.001 M - 0.5 M, preferably at about 0.01 M - 0.25 M, more preferably about 0.05 M - 0.20 M, and most preferably at a concentration of 0.10 M - 0.15 M. The addition may be made in a single or multiple additions, preferably simultaneous with the additions of the intercalating agents.

It may be desirable to remove the unreacted antioxidant and/or singlet oxygen scavenger and/or their photoreaction products from the irradiated sample. This can be readily achieved by one of several standard methods known to a skilled person such as dialysis across an appropriate sized membrane or through an appropriate sized hollow fiber system after completion of the irradiation. Alternatively, a person skilled in the art could use affinity columns for one or more of the low molecular weight compounds to be removed.

In a commercial scale production samples of one to two hundred liters per week can be subjected to the inventive method for adenovirus inactivation and purification as described herein.

After the irradiation step the amount of protein, particularly the amount of enzyme, remaining in its biologically active form is normally higher than 75%, preferably higher than 80%, more preferably higher than 85%, and even more preferably higher than 90%, and particularly higher than 95%.

The particular culture medium which is used for virus production and simultaneous protein production can be a conventional and/or commercially available culture medium, such as Eagle's Minimum Essential Medium or Medium 199, usually supplemented with additives such as broth prepared from dehydrated standard microbial culture media, fetal bovine serum, calf serum, or the like. HEPES buffer may be used to adjust the pH value. Also other appropriate buffers, such as Tris buffer, which will not interfere with the subsequent processing may be used.

As used herein, the term "sample" preferably refers to water-based solutions or suspensions of the above-mentioned components. Also the addition of a cosolvent may be advantageous. The addition of at least one organic solvent may increase the permeability of the outer coat or membrane of the virus. Such increase in permeability would facilitate penetration by the intercalating agent and enhances the inactivation level of the viruses.

Preferred cosolvents which may be used in an amount of up to 30% (v/v) can be selected from the group comprising dimethylsulfoxide (DMSO) ethers, such as dioxane and tetrahydrofurane; ketones, such as acetone and butanone; alcohols, such as methanol, ethanol, propanol, iso-propanol, glycerol, polyethylene glycol (PEG), polypropylene glycol; acids, such as formic acid and acetic acid; acid esters, such as acetic acid methyl ester and acetic acid ethyl ester; and amides, such as dimethylformamide (DMF) and dimethylacetamide. Most preferably DMSO and DMF are used as cosolvents. A cosolvent could increase the inactivation level of the adenoviruses.

The amount of adenovirus will generally be about 1×10⁶ to 10¹⁴ pfu/ml, more usually about 1×10⁷ to 10¹³ pfu/ml, preferably about 1×10⁸ to 5×10¹² pfu/ml, more preferably about 1×10⁹ to 1×10¹² pfu/ml, and most preferably about 5×10⁹ to 5×10¹¹ pfu/ml.

The inactivation level of the virus is normally higher than 2log₁₀, preferably higher than 3log₁₀, more preferably higher than 4log₁₀, and most preferably higher than 4,5log₁₀.

Said samples containing the virus suspension are irradiated with light in the presence of a UV-sensitive intercalating agent preferably in an inert atmosphere for a time sufficient to completely inactivate the virus. The resulting inactivated virus suspension may be stored until purification or for subsequent use.

Within the inventive method any type of adenovirus can be used, directing the synthesis of a desired gene product. The virus could e.g. be an adenovirus type, such as adenovirus type 2 or 5, adenovirus type 3, adenovirus type 7, the avian adenovirus CELO, any other adenovirus, a parvovirus such as adeno-associated virus or chicken adeno-associated virus etc.

Concerning the separation and/or purification step of the inventive method, although examples of the affinity tag Strep-tag are presented here, the method is not limited to this technique. A large variety of affinity tags are available for protein production (reviewed in Constans, A. Protein Purification II: Affinity Tags. The Scientist 16: 37). Furthermore, the target protein can also be purified by standard chromatographic methods, such as ion exchange, size-exclusion, hydrophobic interaction, dye-binding or other chromatographic purification methods.

A preferred embodiment of said method comprises the further step of purifying the biological material by affinity tag ("Strep-tag" technology). Said Strep-tag technology is described in detail in the patents US 5,506,121; UK 2272698; and FR 93 13 066. The tetracycline promotor based expression system is disclosed in US Patent 5,849,576 and Strep-Tactin® is disclosed in the US Patent 6,103,493. Further references which are incorporated herewith by refernce are: 1. Schmidt TG, Skerra A. One-step affinity purification of bacterially produced proteins by means of the "Strep tag" and immobilized recombinant core streptavidin. J Chromatogr A 1994 Aug 5; 676(2):337 to 345; 2. Schmidt TG, Koepke J, Frank R, Skerra A. Molecular interaction between the Strep-tag affinity peptide and its cognate target, streptavidin. J Mol Biol 1996 Feb 9; 255(5): 753 to 766; 3. Skerra A, Schmidt TG. Use of the Strep-Tag and streptavidin for detection and purification of recombinant proteins. Methods Enzymol 2000; 326:271 to 304.

Furthermore, the present invention discloses mammalian proteins which are obtainable by the inventive methods.

As mammalian proteins for example enzymes, lipoproteins, hormones, antibodies, co-factors, growth factors, molecules of blood-clotting cascades, lymphokines, clotting factors, molecules of signal transduction cascades, immunoglobulines, cytokines, molecular chaperones, endorphins nucleases, ribonucleases such as DICER, deoxyribonucleases, and proteases can be mentioned.

Preferred as mammalian proteins are enzymes selected from the group comprising oxidoreductases, transferases, hydrolases, lyases, isomerases, ligases, and especially kinases and phosphatases. Normally, said biologically active materials are non-viral proteins and more preferably non-viral proteins produced recombinantly in a viral system.

More preferably, the mammalian proteins are selected from the group comprising mammalian or human hexokinase, glucokinase, ketohexokinase, fructokinase, rhamnulokinase, galactokinase, mannokinase, glucosamine kinase, phosphoglucokinase, 6-phosphofructokinase, gluconokinase, dehydogluconokinase, sedoheptulokinase, ribokinase, L-ribulokinase, xylulokinase, phosphoribokinase, phosphoribulokinase, adenosine kinase, thymidine kinase, ribosylnicotinamide kinase, NAD(+) kinase, dephospho-CoA kinase, adenylylsulfate kinase, riboflavin kinase, erythritol kinase, triokinase, glycerone kinase, glycerol kinase, glycerate kinase, choline kinase, pantothenate kinase, pantetheine kinase, pyridoxal kinase, mevalonate kinase, protein kinase, phosphorylase kinase, homoserine kinase, pyruvate kinase, glucose-1-phosphate phosphodismutase, riboflavin phosphotransferase, glucuronokinase, galacturonokinase, 2-dehydro-3-deoxygluconokinase, L-arabinokinase, D-ribulokinase, uridine kinase, hydroxymethylpyrimidine kinase, hydroxyethylthiazole kinase, L-fuculokinase, fucokinase, L-xylulokinase, D-arabinokinase, allose kinase, 1-phosphofructokinase, 2-dehydro-3-deoxygalactonokinase, N-acetylglucosamine kinase, N-acylmannosamine kinase, acyl-phosphate-hexose phosphotransferase, phosphoramidate-hexose phosphotransferase, polyphosphate-glucose phosphotransferase, myo-inositol 1-kinase, scyllo-inosamine kinase, undecaprenol kinase, 1-phosphatidylinositol 4-kinase, 1-phosphatidylinositol-4-phosphate kinase, protein-N(Pl)-phosphohistidine-sugar phosphotransferase, protamine kinase, shikimate kinase, streptomycin 6-kinase, inosine kinase, deoxycytidine kinase, deoxyadenosine kinase, nucleoside phosphotransferase, polynucleotide 5'-hydroxyl-kinase, diphosphate--glycerol phosphotransferase, diphosphate--serine phosphotransferase, hydroxylysine kinase, ethanolamine kinase, pseudouridine kinase, alkylglycerone kinase, beta-glucoside kinase, NADH kinase, streptomycin 3"-kinase,dihydrostreptomycin-6-phosphate 3'-alpha-kinase, thiamine kinase, diphosphate--fructose-6-phosphate 1-phosphotransferase, sphinganine kinase, 5-dehydro-2-deoxygluconokinase, alkylglycerol kinase, acylglycerol kinase, kanamycin kinase, [pyruvate dehydrogenase(lipoamide)] kinase, 5-methylthioribose kinase, tagatose kinase, hamamelose kinase, viomycin kinase, diphosphate-protein phosphotransferase, 6-phosphofructo-2-kinase, glucose-1,6-bisphosphate synthase, diacylglycerol kinase, dolichol kinase, [hydroxymethylglutaryl-CoA reductase(NADPH)] kinase, dephospho-[reductase kinase] kinase, protein-tyrosine kinase, deoxyguanosine kinase, AMP--thymidine kinase, [3-methyl-2-oxobutanoate dehydrogenase (lipoamide)] kinase, [isocitrate dehydrogenase (NADP+)] kinase, [myosin light-chain] kinase, ADP-thymidine kinase, hygromycin-B kinase, caldesmon kinase, phosphoenolpyruvate--glycerone phosphotransferase, xylitol kinase, calcium/calmodulin-dependent protein kinase, tyrosine 3-monooxygenase kinase, rhodopsin kinase, [beta-adrenergic-receptor] kinase, 1D-myo-inositol-trisphosphate 3-kinase, [acetyl-CoA carboxylase] kinase, [myosin heavy-chain] kinase, tetraacyldisaccharide 4'-kinase, [low-density lipoprotein receptor] kinase, tropomyosin kinase, 1D-myo-inositol-trisphosphate 6-kinase, 1D-myo-inositol-tetrakisphosphate 1-kinase, [tau protein] kinase, macrolide 2'-kinase, 1-phosphatidylinositol 3-kinase, ceramide kinase, 1D-myo-inositol-trisphosphate 5-kinase, 1D-myo-inositol-tetrakisphosphate 5-kinase, [RNA-polymerase]-subunit kinase, glycerol-3-phosphate-glucose phosphotransferase, diphosphate-purine nucleoside kinase, tagatose-6-phosphate kinase, acetate kinase, carbamate kinase, phosphoglycerate kinase, aspartate kinase, formate kinase, butyrate kinase, acetylglutamate kinase, phosphoglycerate kinase (GTP), glutamate 5-kinase, acetate kinase (diphosphate), glutamate 1-kinase, branched-chain-fatty-acid kinase, guanidoacetate kinase, creatine kinase, arginine kinase, taurocyamine kinase, lombricine kinase, hypotaurocyamine kinase, opheline kinase, ammonia kinase, phosphoenolpyruvate--protein phosphatase, agmatine kinase, protein-histidine pros-kinase, protein-histidine tele-kinase, polyphosphate kinase, phosphomevalonate kinase, adenylate kinase, nucleoside-phosphate kinase, nucleoside-diphosphate kinase, phosphomethylpyrimidine kinase, guanylate kinase, thymidylate kinase, nucleoside-triphosphate--adenylate kinase, (deoxy)adenylate kinase, T2-induced deoxynucleotide kinase, (deoxy)nucleoside-phosphate kinase, cytidylate kinase, thiamine-diphosphate kinase, thiamine-phosphate kinase, 3-phosphoglyceroyl-phosphate-polyphosphate phosphotransferase, farnesyl-diphosphate kinase, 5-methyldeoxycytidine-5'-phosphate kinase, dolichyl-diphosphate--polyphosphate phosphotransferase, ribose-phosphate pyrophosphokinase, thiamine pyrophosphokinase, 2-amino-4-hydroxy-6-hydroxymethyldihydropteridine pyrophosphokinase, nucleotide pyrophosphokinase, GTP pyrophosphokinase, nicotinamide-nucleotide adenylyltransferase, FMN adenylyltransferase, pantetheine-phosphate adenylyltransferase, sulfate adenylyltransferase, sulfate adenylyltransferase (ADP), DNA-directed RNA polymerase, DNA-directed DNA polymerase, polyribonucleotide nucleotidyltransferase, UTP--glucose-1-phosphate uridylyltransferase, UTP--hexose-1-phosphate uridylyltransferase, UTP--xylose-1-phosphate uridylyltransferase, UDP-glucose--hexose-1-phosphate uridylyltransferase, mannose-1-phosphate guanylyltransferase, ethanolamine-phosphate cytidylyltransferase, cholinephosphate cytidylyltransferase, nicotinate-nucleotide adenylyltransferase, polynucleotide adenylyltransferase, tRNA cytidylyltransferase, mannose-1-phosphate guanylyltransferase (GDP), UDP-N-acetylglucosamine pyrophosphorylase, glucose-1-phosphate thymidylyltransferase, tRNA adenylyltransferase, glucose-1-phosphate adenylyltransferase, nucleoside-triphosphate-hexose-1-phosphate nucleotidyltransferase, hexose-1-phosphate guanylyltransferase, fucose-1-phosphate guanylyltransferase, DNA nucleotidylexotransferase, galactose-1-phosphate thymidylyltransferase, glucose-1-phosphate cytidylyltransferase, glucose-1-phosphate guanylyltransferase, ribose-5-phosphate adenylyltransferase, aldose-1-phosphate adenylyltransferase, aldose-1-phosphate nucleotidyltransferase, 3-deoxy-manno-octulosonate cytidylyltransferase, glycerol-3-phosphate cytidylyltransferase, D-ribitol-5-phosphate cytidylyltransferase, phosphatidate cytidylyltransferase, glutamate-ammonia-ligase adenylyltransferase, acylneuraminate cytidylyltransferase, glucuronate-1-phosphate uridylyltransferase, guanosine-triphosphate guanylyltransferase, gentamicin 2"-nucleotidyltransferase, streptomycin 3"-adenylyltransferase, RNA-directed RNA polymerase, RNA-directed DNA polymerase, mRNA guanylyltransferase, adenylylsulfate--ammonia adenylyltransferase, RNA uridylyltransferase, ATP adenylyltransferase, phenylalanine adenylyltransferase, anthranilate adenylyltransferase, tRNA nucleotidyltransferase, N-methylphosphoethanolamine cytidylyltransferase, (2,3-dihydroxybenzoyl)adenylate synthase, [protein-PII] uridylyltransferase, ethanolaminephosphotransferase, diacylglycerol cholinephosphotransferase, ceramide cholinephosphotransferase, serine-phosphoethanolamine synthase, CDP-diacylglycerol--glycerol-3-phosphate 3-phosphatidyltransferase, undecaprenyl-phosphate galactosephosphotransferase, holo-[acyl-carrier protein] synthase, CDP-diacylglycerol--serine O-phosphatidyltransferase, phosphomannan mannosephosphotransferase, sphingosine cholinephosphotransferase, CDP-diacylglycerol--inositol 3-phosphatidyltransferase, CDP-glycerol glycerophosphotransferase, phospho-N-acetylmuramoyl-pentapeptide-transferase, CDP-ribitol ribitolphosphotransferase, UDP-N-acetylglucosamine--dolichyl-phosphate (N-acetylglucosaminephosphotransferase), UDP-N-acetylglucosamine--lysosomal-enzyme, (N-acetylglucosaminephosphotransferase), UDP-galactose--UDP-N-acetylglucosamine galactosephosphotransferase, UDP-glucose--glycoprotein glucosephosphotransferase, phosphatidylglycerol--membrane-oligosaccharide glycerophosphotransferase, membrane-oligosaccharide glycerophosphotransferase, 1-alkenyl-2-acylglycerol cholinephosphotransferase, carboxyvinyl-carboxyphosphonate phosphorylmutase, pyruvate-phosphate dikinase, Pyruvate-water dikinase, Selenide-water dikinase.

Of special interest is the generation of mammalian kinases, preferably human kinases. These enzymes form the basis of the mammalian signalling network which controls the cellular response to the environment. Efforts to modulate signal transduction pathways require quantities of active kinases in relatively pure form (i.e., free of any other kinase activity).

Most preferred is the production of mammalian kinases, such as cdk9/cyclin T1 and RICK (Rip2, Cardiak). A total of more than 500 mammalian kinases are known (Manning G, Whyte DB, Martinez R, Hunter T, Sudarsanam S; The protein kinase complement of the human genome, Science 2002 Dec 6; 298 (5600):1912 to 1934). By now, there is no clue or indication that one of the following mammalian kinases listed below cannot be produced by means of the inventive method presented herein. An important point is that these kinases can be used for cosmetic and/or medical and/or analytical purposes, because no active viruses, especially adenoviruses, can be detected in said biological material, i.e. the biological material (such as kinases) is not contaminated with viruses in a replicable form.
KIAA0472 protein, SgK496
KIAA0561 protein, MAST3
STK38L
SAST (syntrophin associated serine/threonine kinase)
QSK, KIAA0999 protein
TTBK1
MAP3K1; MEKK1
IKK-beta; IKK2
LATS2 (LATS, large tumor suppressor, homolog 2)
SNRK (SNF-1 related kinase)
MAP3K9; MLK1
GRK7 (G protein-coupled receptor kinase 7)
STK22C; TSSK3
similar to serologically defined breast cancer antigen NY-BR-96
MYO3B
PRKWNK4 (protein kinase, lysine deficient 4)
PXK (PX domain-containing protein kinase)
PRKWNK3 (protein kinase, lysine deficient 3)
hypothetical protein MGC4796
KSR (kinase suppressor of ras)
PIM3
KIAA1639
putative serine/threonine kinase SgK495
DCAMKL2
SPEG, KIAA1297 protein
ortholog to ratERK7; ERK8
NRBP2
TSSK4
CLIK1L
ULK3
SgK493
MAST4, KIAA0303 protein
similar to Ca2+/Calmodulin-dependent protein kinase I
similar to cyclin-dependent kinase-like 1; serine/threonine protein kinase KKIALRE
similar to protein kinase related to Raf
ortholog to rat sbk
YANK1
LOC120392
SCYL2
SNF1 LK
MLKL, LOC146593
NEK10
hypothetical protein DKFZp761 P0423
hypothetical protein FLJ21140
LRRK1, hypothetical proteinFLJ23119
TBCK, hypothetical portein MGC16169
SgK424, similar to testis expressed gene 14 (LOC126392)
TEX14 (testis expressed sequence 14)
TTBK2
MAP3K7
NEK5
similar to MLCK
DCAMKL3, KIAA1765 protein
MAP3K8
NRK/ZC4 (NIK-related kinase)
Wee1B
SgK494
PRKWNK2 (protein kinase, lysine deficient 2)
KIAA1028 protein
STK35
C20orf97 (chromosome 20 open reading frame 97)
ACVRL1 (activin A receptor type II-like 1)
CDK4 (cyclin-dependent kinase 4)
PHKG2 (phosphorylase kinase, gamma 2)
STK11; LKB1
ACVR1 (activin A receptor, type I)
ACVR2B, activin A receptor, type IIB
BMPR1B (bone morphogenetic protein receptor, type IB)
BMPR2 (bone morphogenetic protein receptor, type II (serine/threonine kinase)) BUB1B (BUB1 budding uninhibited by benzimidazoles 1 homolog beta)
CAMK2B (calcium/calmodulin-dependent protein kinase (CaM kinase) II beta)
CAMK2D (calcium/calmodulin-dependent protein kinase (CaM kinase) II delta)
CDK3 (cyclin-dependent kinase 3)
CDK6 (cyclin-dependent kinase 6)
CDK8 (cyclin-dependent kinase 8)
CDK9 (cyclin-dependent kinase 9 (CDC2-related kinase))
CHEK1 (CHK1 checkpoint homolog)
IKK-alpha; CHUK
MAPK14; CSBP1
CSNK1G2 (casein kinase 1, gamma 2)
DAPK3 (death-associated protein kinase 3)
DYRK1A (dual-specificity tyrosine-(Y)-phosphorylation regulated kinase 1A)
ERN1 (ER to nucleus signalling 1)
IRAK1 (interleukin-1 receptor-associated kinase 1)
IRAK2 (interleukin-1 receptor-associated kinase 2)
ACVR2, activin A receptor, type II
GRK2
AKT2 (v-akt murine thymoma viral oncogene homolog 2)
ARAF1 (v-raf murine sarcoma 3611 viral oncogene homolog 1)
CAMK4 (calcium/calmodulin-dependent protein kinase IV)
CDC2 (cell division cycle 2)
CDC2L1
CDK2 (cyclin-dependent kinase 2)
CDK7 (cyclin-dependent kinase 7)
CSNK1A1 (casein kinase 1, alpha 1)
CSNK1D (casein kinase 1, delta)
CSNK1 E (casein kinase 1, epsilon)
CSNK2A1 (casein kinase 2, alpha 1)
CSNK2A2 (casein kinase 2, alpha prime)
GRK6
GSK3B (glycogen synthase kinase 3 beta)
LIMK1 (LIM domain kinase 1)
MARK3 (MAP/microtubule affinity-regulating kinase 3)
MAP3K3; MEKK3
MAP3K11; MLK3
MAP3K10; MST; MLK2
NEK2 (NIMA (never in mitosis gene a)-related kinase 2)
NEK3 (NIMA (never in mitosis gene a)-related kinase 3)
PAK1
PAK2
PAK3
PCTK2 (PCTAIRE protein kinase 2)
PDK1 (pyruvate dehydrogenase kinase, isoenzyme 1)
PDK2 (pyruvate dehydrogenase kinase, isoenzyme 2)
PDPK1 (3-phosphoinositide dependent protein kinase-1)
PIM1
PIK3CG (phosphoinositide-3-kinase, catalytic, gamma polypeptide)
PRKACA (protein kinase, cAMP-dependent, alpha)
PRKACB (protein kinase, cAMP-dependent, beta)
PRKACG (protein kinase, cAMP-dependent, gamma)
PRKCA (protein kinase C, alpha)
PRKCB1 (protein kinase C, beta 1)
PRKCG (protein kinase C, gamma)
PRKCI (protein kinase C, iota)
PRKCL1 (protein kinase C-like 1)
PRKCM (protein kinase C, mu)
PRKCZ (protein kinase C, zeta)
MAPK1, ERK2
MAPK4; ERK3
MAPK6; ERK3
MAPK7; ERK5
MAPK8; JNK1
MAPK11; p38beta
MAPK9; JNK2
MAPK10; JNK3
MAPK13; p38delta
MAP2K1; MEK1
MAP2K3; MEK3
MAP2K5; MEK5
MAP2K6; MEK6
PRKR (protein kinase, interferon-inducible double stranded RNA dependent)
PRKY (protein kinase, Y-linked)
RAF1 (v-raf-1 murine leukemia viral oncogene homolog 1)
GRK1; rhodopsin kinase
RPS6KA1 = ribosomal protein S6 kinase, 90kD, polypeptide 1
MAPK12; ERK6
MAP2K4; MEK4
SRPK1 (SFRS protein kinase 1)
SRPK2 (SFRS protein kinase 2)
STK2
STK9
STK13; (aurora/IPL1-like)
RPS6KB1 (ribosomal protein S6 kinase, 70kD, polypeptide 1)
MAP3K7; TAK1
TGFBR2 (transforming growth factor, beta receptor II)
TTK protein kinase
Titin
VRK1 (vaccinia related kinase 1)
WEE1
CDC7L1 (CDC7 cell division cycle 7-like 1)
ULK1 (unc-51-like kinase 1)
STK24; MST3
DYRK3 (dual-specificity tyrosine-(Y)-phosphorylation regulated kinase 3)
DYRK2 (dual-specificity tyrosine-(Y)-phosphorylation regulated kinase 2)
STK15; BTAK
PK428
MAP4K3; GLK
CAMK1 (calcium/calmodulin-dependent protein kinase I)
MAPKAPK5
CDK10 (cyclin-dependent kinase (CDC2-like) 10)
MKNK1 (MAP kinase-interacting serine/threonine kinase 1); MNK1
CASK (calcium/calmodulin-dependent serine protein kinase (MAGUK family))
STK16; PKL12
CDC2L5 (cell division cycle 2-like 5)
RIPK1 (receptor (TNFRSF)-interacting serine-threonine kinase 1); RIP
RIPK2 (receptor-interacting serine-threonine kinase 2);
RICK
PRP4
RPS6KA4 (ribosomal protein S6 kinase, 90kD, polypeptide 4); MSK2
CDKL2 (cyclin-dependent kinase-like 2); KKIAMRE
RPS6KB2 (ribosomal protein S6 kinase, 70kD, polypeptide 2)
MAP3K14; NIK
CLK3 (CDC-like kinase 3)
CLK2 (CDC-like kinase 2)
CLK1 (CDC-like kinase 1)
CNK (cytokine-inducible kinase)
CDKL1 (cyclin-dependent kinase-like 1);
KKIALRE
PKMYT1
STK12; IPL1
STK17B; DRAK2
ACVR1B (activin A receptor, type IB)
BMPR1A (bone morphogenetic protein receptor, type IA)
BRAF (v-raf murine sarcoma viral oncogene homolog B1)
BUB1 (BUB1 budding uninhibited by benzimidazoles 1 homolog)
CSNK1G3 (casein kinase 1, gamma 3)
DMPK (dystrophia myotonica-protein kinase)
ILK (integrin-linked kinase)
MAP4K2; GCK
RPS6KA3 = ribosomal protein S6 kinase, 90kD, polypeptide 3; RSK2
TGFBR1 (transforming growth factor, beta receptor I (activin A receptor type II-like kinase, 53kD))
MAPKAPK3
MAP3K6
LATS1 (LATS, large tumor suppressor, homolog 1)
DYRK1B (dual-specificity tyrosine-(Y)-phosphorylation regulated kinase 1B)
MAP3K13; LZK
DCAMKL1 (doublecortin and CaM kinase-like 1)
RPS6KA5 (ribosomal protein S6 kinase, 90kD, polypeptide 5); MSK1
MAPKAPK2
STK17A; DRAK1
MAP4K4; NIK; HGK
EIF2AK3 (eukaryotic translation initiation factor 2-alpha kinase 3)
ROCK2 (Rho-associated, coiled-coil containing protein kinase 2)
CDK5 (cyclin-dependent kinase 5)
DAPK1 (death-associated protein kinase 1)
EMK1 (ELKL motif kinase)
PIK3CD (phosphoinositide-3-kinase, catalytic, delta polypeptide
PLK (polo-like kinase)
MAP2K7; MKK7
PRKX (protein kinase, X-linked)
OSR1 (oxidative-stress responsive 1)
GRK3
AKT1 (v-akt murine thymoma viral oncogene homolog 1)
MAP3K8; Tpl-2
GAK (cyclin G associated kinase)
GRK4
GRK5
MOS (v-mos Moloney murine sarcoma viral oncogene homolog)
PRKCE (protein kinase C, epsilon)
ROCK1 (Rho-associated, coiled-coil containing protein kinase 1); p160ROCK
AKT3 (v-akt murine thymoma viral oncogene homolog 3 (protein kinase B, gamma))
LIMK2 (LIM domain kinase 2)
SGK (serum/glucocorticoid regulated kinase)
HIPK3 (homeodomain interacting protein kinase 3)
PRKCN (protein kinase C, nu)
BCKDK (branched chain alpha-ketoacid dehydrogenase kinase)
PAK4
MAK (male germ cell-associated kinase)
MAP3K4; MEKK4
MAP3K5; ASK1
MYLK (myosin, light polypeptide kinase)
STK10; LOK
CDC42BPB (CDC42 binding protein kinase beta (DMPK-like))
PCTK1 (PCTAIRE protein kinase 1)
PHKG1 (phosphorylase kinase, gamma 1)
PIK3CA (phosphoinositide-3-kinase, catalytic, alpha polypeptide)
PIK3CB (phosphoinositide-3-kinase, catalytic, beta polypeptide)
PRKAA1 (protein kinase, AMP-activated, alpha 1 catalytic subunit)
PRKAA2 (protein kinase, AMP-activated, alpha 2 catalytic subunit)
PRKCD (protein kinase C, delta)
PRKCH (protein kinase C, eta)
PRKCL2 (protein kinase C-like 2)
PRKCQ (protein kinase C, theta)
PRKG1 (protein kinase, cGMP-dependent, type I)
PRKG2 (protein kinase, cGMP-dependent, type II); cGKll
STK3; MST2
STK4; MST1
TESK1 (testis-specific kinase 1)
VRK2 (vaccinia related kinase 2)
MAP3K12; DLK
STK25; YSK1
HTATIP2 (HIV-1 Tat interactive protein 2, 30 kD)
CAMKK2 (calcium/calmodulin-dependent protein kinase kinase 2, beta)
MAP4K5
MAP3K2; MEKK2
SNK (serum-inducible kinase)
TLK2 (tousled-like kinase 2)
RIPK3 (receptor-interacting serine-threonine kinase 3);
RIP3
PIM2
PRKDC (protein kinase, DNA-activated)
TRAD
TRIO (triple functional domain (PTPRF
interacting))
TESK2 (testis-specific kinase 2)
MAP4K1; HPK1
CHEK2 (CHK2 checkpoint homolog)
IRAK-M
STK38; NDR
CCRK (cell cycle related kinase)
TLK1 (tousled-like kinase 1)
PFTK1 (PFTAIRE protein kinase 1)
RPS6KC1 (ribosomal protein S6 kinase, 52kD, polypeptide 1)
STK39; SPAK
TBK1 (TANK-binding kinase 1)
SGKL (serum/glucocorticoid regulated kinase-like)
PKNbeta
NRBP (nuclear receptor binding protein
IKKE (IKK-related kinase epsilon; inducible IkappaB kinase)
SMG1
ITPK1 (inositol 1,3,4-triphosphate 5/6 kinase)
RAGE1 (renal tumor antigen)
STK18; Sak
DAPK2 (death-associated protein kinase 2)
protein kinase H11
STK23; MSSK1
NEK6 (NIMA (never in mitosis gene a)-related kinase 6)
HRI (heme-regulated initiation factor 2-alpha kinase)
RPS6KA6 (ribosomal protein S6 kinase, 90kD, polypeptide 6); RSK4 HUNK (hormonally upregulated Neu-associated kinase)
PIK3R4 (phosphoinositide-3-kinase, regulatory subunit 4, p150)
ULK2 (unc-51-like kinase 2)
SLK (SNF1 sucrose nonfermenting like kinase)
KIAA0175
KIAA0537 gene product
AAK1
KIAA1079 protein
MAK-related kinase
MAST205
PASK (PAS domain containing serine/threonine kinase)
STK36
MINK (Misshapen/NIK-related kinase)
CAMK2A (calcium/calmodulin-dependent protein kinase (CaM kinase)II alpha)
IRAK4
PSK
NLK
SGK2 (serum/glucocorticoid regulated kinase 2)
JIK
VRK3 for vaccinia related kinase 3
PKD2 (polycystic kidney disease 2)
CrkRS
CDKL3 (cyclin-dependent kinase-like 3); NKIAMRE
MST4
ZAK (sterile-alpha motif and leucine zipper containing kinase AZK)
MYO3A
GPRK7 (G protein-coupled receptor kinase 7)
BIKE (homolog of mouse BMP-2 inducible kinase)
hypothetical protein FLJ20574
serine/thronine kinase HSA250839
TOPK (T-LAK cell-originated protein kinase)
ALS2CR2 (amyotrophic lateral sclerosis 2 (juvenile) chromosome region, candidate 2)
MARK1 (MAP/microtubule affinity-regulating kinase 1)
PRKWNK1 (protein kinase, lysine deficient 1); WNK1
GSK3A (glycogen synthase kinase 3 alpha)
PAK6
PAK5 (PAK7)
CKLiK
PACE-1
CAMK1G (calcium/calmodulin-dependent protein kinase IG)
AMHR2 (anti-Mullerian hormone receptor, type II)
ANKRD3 (ankyrin repeat domain 3); DIK
CLK4 (CDC-like kinase 4)
NTKL (N-terminal kinase-like)
RNASEL (ribonuclease L (2',5'-oligoisoadenylate synthetase-dependent))
RPS6KA2 (ribosomal protein S6 kinase, 90kD, polypeptide 2); RSK3
BCR1
GS3955 protein
CSNK1G1 (casein kinase 1, gamma 1)
HIPK2 (homeodomain interacting protein kinase 2)
hypothetical protein MGC8407
NEK11
FASTK (Fas-activated protein kinase)
C8FW
MAP2K2; MEK2
hypothetical protein DKFZp434J037
STK31
MARKL1 (MAP/microtubule affinity-regulating kinase like 1)
hypothetical protein MGC11287 similar to ribosomal protein S6 kinase
GSG2
FKSG81
SSTK
hypothetical protein FLJ23356
hypothetical protein DKFZp761M0423
PINK1 (PTEN induced putative protein kinase 1)
BRSK1, KIAA1811
MASTL, hypothetical protein FLJ14813
NIMA-related kinase Nek8, NEK9
MYLK2 (myosin light chain kinase 2)
PSKH2
ERN2 (ER to nucleus signalling 2)
CDC2L1 (cell division cycle 2-like 1); PITSLRE
PRPK
STK22B; TSSK2
NEK7
ALS2CR7
KIS
HIPK4
CKla2
ALK7
Nbak2, KIAA0630 protein
MGC42105, similar to serine/threonine kinase (KlN1/SNF1/Nim1 subfamily)
YANK3
NEK12A, NEK8
MAPK3;ERK1
GCN2, elF2alpha kinase
STK33
DYRK4 (dual-specificity tyrosine-(Y)-phosphorylation regulated kinase 4)
TNIK (Traf2 and NCK interacting kinase)
similar to myosin light chain kinase (MLCK)
KIAA0781
PSKH1 (protein serine kinase H1)
CAMK2G (calcium/calmodulin-dependent protein kinase (CaM kinase) II gamma)
Citron
PCTK3 (PCTAIRE protein kinase 3)
similar to Ser/Thr protein kinase PAR-1 alpha
STK29; PEN11B
similar to protein kinase Bsk146
KIAA1361
NEK1 (NIMA (never in mitosis gene a)-related kinase 1)
MGC43306, SgK071
myotonic dystrophy protein kinase like protein
ILK-2
MLK4beta
EphA9
SGK040
SGK007
insulin receptor-related receptor, partial sequence
ZAP-70
TTK
BTK
FGFR-2
FGFR-3
GUCY2D
Insulin receptor
JAK-3
c-Kit
Met protooncogene
TEK
FGFR-1
insulin-like growth factor 1 receptor
natriuretic peptide receptor A/guanylate cyclase A
natriuretic peptide receptor B/guanylate cyclase B
guanylate cyclase 2F, retinal
BLK (B lymphoid tyrosine kinase)
BMX
ERBB3
FES (feline sarcoma oncogene)
FGFR-4
FLT-1
FLT-4
FRK (fyn-related kinase)
FYN
HCK
JAK-1
KDR kinase insert domain receptor
LTK
LYN
MATK (megakaryocyte-associated tyrosine kinase)
MST1 R (macrophage stimulating 1 receptor)
NTRK1 (neurotrophic tyrosine kinase, receptor, type 1)
NTRK3 (neurotrophic tyrosine kinase, receptor, type 3)
PDGFRB
PTK7
PTK9
ROS1
RYK
SYK
TEC
TXK
TYK2
TNK1
PTK2B
FLT-3
ALK (anaplastic lymphoma kinase (Ki-1))
CSK
EPHA2
EPHA4
EPHA7
EPHB1
EPHB2
EPHB3
EPHB4
EPHB6
ERBB2
ROR2
AATK (apoptosis-associated tyrosine kinase)
GUCY2C
JAK-2
ROR1
ABL1
ABL2
CSF1 R
EGFR
EPHA1
EPHA3
ERBB4
FER
FGR
LCK
SRC
TIE
YES1
ITK
MUSK
PTK2, FAK
ACK1
PTK6
NTRK2 (neurotrophic tyrosine kinase, receptor, type 2)
DDR2
PDGFRA
TYRO3
MERTK (c-mer proto-oncogene tyrosine kinase)
PTK9L
DDR1
LOC51086 (putative protein-tyrosine kinase)
DKFZp761 P1010 (hypothetical protein DKFZp761 P1010)
EPHA8
RET
AXL
TRK-T3
EPHA5
EPHA6
KIAA1883
SGK119
G11
BRDT
BRD2
BRD3
BRD4
TAF1
TAF1 L
TIF1A
TIF1B
TIF1G
ADCK1
ADCK2
ADCK3
ADCK4
ADCK5
PDK3
PDK4
EF2 Kinase
Lak
TRPM6
HAK
MIDORI
FRAP1
phosphoinositide-3 kinase
ATR
phosphatidylinositol 3-kinase
phosphatidylinositol 4-kinase NPIK-C
phosphatidylinositol 4-kinase 230
TRRAP
phosphoinositide 3-kinase beta
phosphatidylinositol 3-kinase gamma
ATM
RIOK1
RIOK2
RIOK3

Similarly, it is preferred to produce one of the following phosphatases with the method according to the present invention:
DUSP5
BDP-1
CD45
DUSP1
PTPRJ
PTPRO
PTPN1
KAP-1
LAR
LC-PTP
Pac-1
PTPRU
Pez
PP1-Calpha
PP1-Cbeta
PP1-Cgamma
PP2A-Calpha
PP2A-Cbeta
PP2B-Calpha
PP2B-Cbeta
PP2B-Cgamma
PP2B-R
PP2C
PP2C-alpha
DUSP9
PP5
PP6
PPP4C
Prl-1
PTEN
PTPN13
PTP-D1
PTP-H1
PTPN4
PTPN9
PTP-Pest
PTP-RQ
PTP-SL
PTP-Step
PTPalpha
PTPbeta
PTPdelta
PTPepsilon
PTPgamma
PTPkappa
PTPmu
PTPsigma
PTPzeta
Sap-1
Shp-1
Shp-2
TC-PTP
TRAP
DUSP4
DUSP8
VHR
ACP1
TYP
PP7
DUSP6
PYST1
PPEF-2s
CDC14A1
CDC14B
SBF1
CDC25B
PP2C-beta
FCP1
PRL-3
PIR1
MYPT2
Lyp
LAFPTP1
MKP-L
KIAA0371
MKP-5
PTP4A1
DUSP12
PPM1D
SGP014
PTPD
PTP-rho
PTPLA
FYVE-DSP2
STP
MKPX
ILKAP
PTPN
PTPRN2
C22orf1
C11orf8
MRE11A
Q9BU58
TPTE
MTR7
MTR6
MTR2
MTR1
MYOTUBULARIN
MCE1
MKP-7
KIAA1725
Q9BQ20
Q9BV47
Tensin
Q9H1R2
Q9NXP6
KIAA1298
KIAA1274
KIAA1075
HD-PTP
hypothetical protein
FLJ20442
hypothetical protein
(LOC91443)
hypothetical protein
(LOC63904)
SGP018
LOC118879 sim to protein
phosphatase
SGP040
pyruvate dehydrogenase phosphatase
DKFZp434l1021_r
1
SGP024
Q96CR2
PP 1G
MK-STYX
SKRP1
PP similar to protein phosphatase type 1B
(formely 2C)
Hypothetical protein
FLJ35545
STYX
PTP9Q22

It is demonstrated here that with appropriate optimization, standard, first generation E1-deficient recombinant adenovirus vectors can serve as a useful source of recombinant protein for biochemical purification. An affinity tag can be included on the recombinant protein to facilitate purification. The optimized system can provide 0.5 to 2 mg of pure, active material, for instance active kinase, starting from 18x185 cm² flasks of 293 cells. Treatment of recombinant material preparations with an UV sensitive intercalating agent, such as a psoralen derivative, for instance 8-methoxy psoralen, combined with exposure to long wave UV light completely eliminates residual replicating adenovirus in the preparation. Most importantly, this treatment is gentle to proteins and other biological material such as hormones and no decline in recombinant enzyme activity occurs with this method.

### Brief Description of the Drawings

- Fig. 1: shows optimization of adenovirus infection.
- Fig. 2: shows analysis of RICK extraction.
- Fig. 3: shows purification of RICKdC by classical chromatographic methods.
- Fig. 4: shows production and purification 5 mammalian kinases.
- Fig. 5: shows samples of the purified forms of 5 mammalian kinases.
- Fig. 6: shows the kinase activity of wildtype vs. kinase mutant forms of purified RICK and RIP kinase domains.
- Fig. 7: shows the co-purifying proteins found in RICK and RIP kinase preparations.
- Fig. 8: shows the kinase activity of wildtype vs. kinase mutant forms of cdk9/cyclin T1 complexes.
- Fig. 9: shows the map kinase p38α is produced in an already active form using the inventive system.
- Fig. 10: shows cytopathic effect assay of replicating adenovirus content before and after psoralen/UV treatment.
- Fig. 11: shows RICK kinase activity is not impaired by psoralen/UV treatment.

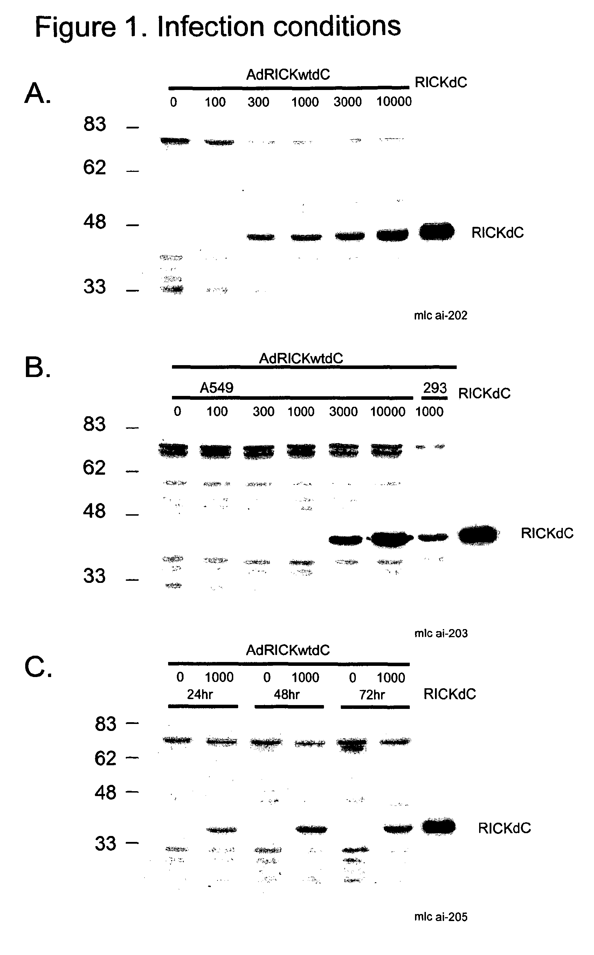

As mentioned above, Fig. 1 shows optimization of adenovirus infection. AdRICKdCst was used to infect 293 or A549 cells (400,000 cells per well, 6 well plate) as indicated below. At 48 hours post infection (panels A and B) or 24, 48 or 72 hours post infection (panel C) the cells were harvested, washed twice with HBS (150 mM NaCl, 20 mM HEPES, pH 7.5) and lysed in 400 µl of SDS application buffer. The samples were heated at 99 C for 10 minutes, sonicated for 5 minutes in a sonicating water bath and 20 µl were loaded per well on a 10%acrylamide SDS gel. After resolution, the gel was transferrred to nitrocellulose, blocked with NET/gelatin, probed with streptavidin-horseradish peroxidase (Amersham) and visualized by ECL.

Panel A: 293 cells infected with 0, 100, 300, 1000, 3000, 10,000 virus particles /cell. RICKdCst. RICKdC indicates an aliquot of streptavidin-Sepharose purified RICKdC as a control. The molecular weights of size standards are indicated to the left of the panel. Panel B: A59 cells infected with 0, 100, 300, 1000, 3000, 10,000 virus particles /cell. For comparison, 293 cell lysate i s the lysate from 293 cells after infection with 1000 particles/cell. Pure RICKdC and molecular weight standards are as in Panel A. Panel C: 293 cells, either uninfected, or infected with 1000 particles/cell AdRICKdCst. Cells were harvested at the indicated times post infection. Pure RICKdC and molecular weight standards are as in Panel A.

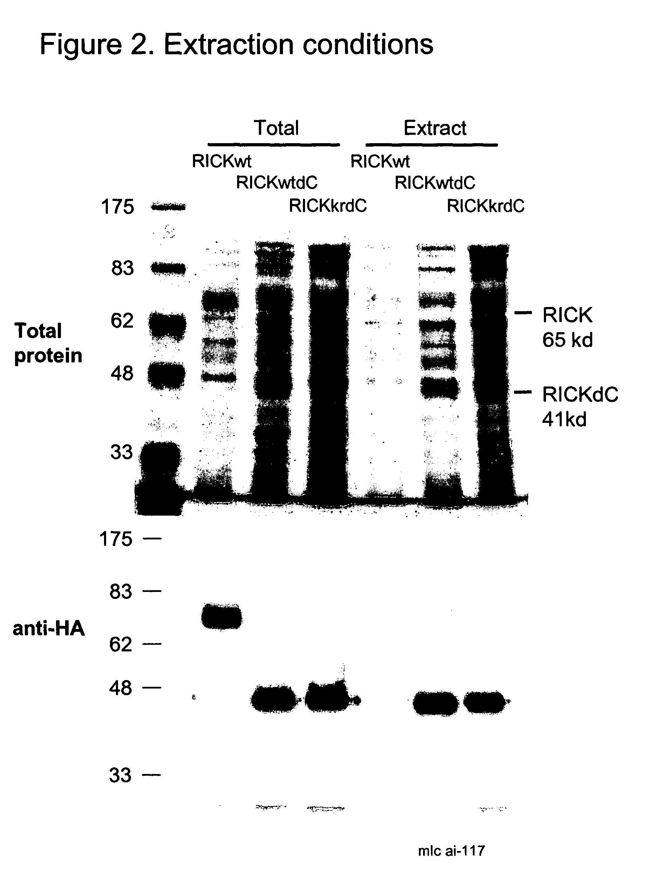

Fig. 2 shows analysis of RICK extraction. 293 cells (80 % confluent 250 cm2 flask) were infected with 1000 particles/ cell AdRICK (full length RICK bearing a carboxy-terminal HA epitope tag) or AdRICKdC (RICK with CARD domain deleted, bearing a carboxy terminal HA tag) with either a wildtype kinase domain (wt) or lysine to arginine, kinase destroying mutation (kr). At 48 hours post-infection, cells were harvested, washed twice with HBS and lysed in 2ml/flask lysis buffer 4 as described in methods section. After lysis, and sonication, an aliquot of the material was saved (Total) the samples were centrifuged at 3300 RPM (Heraeus) for 10 minutes and the supernatant was harvested (Extract). 20 µl aliquots were 20 µl were loaded per well on a 10%acrylamide SDS gel, transferrred to nitrocellulose, stained with Ponceau S to visualize total protein (upper panel), blocked with NET/gelatin, probed with an anti-HA-horseradish peroxidase conjugate (Santa Cruz) and visualized by ECL (bottom panel).

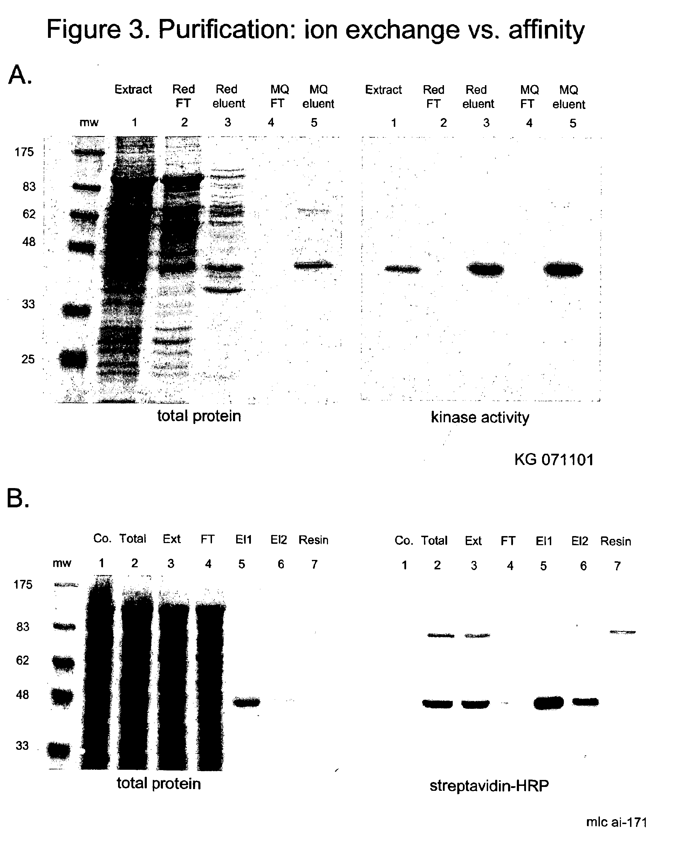

Fig. 3 shows the purification of RICKdC by classical chromatographic methods. RICKwtdC was expressed in 293 cells (infection with AdRICKwtdC at 1000 particles/cell, harvest at 48 hours, lysis in lysis buffer 5 at 2 ml/250 cm² flask equivalent and subjected to chromatographic purification as described in Methods). A typical fractionation pattern is shown with Coomassie blue staining (total protein, left panel) and RICK autophosphorylation activity (kinase activity, right panel). The molecular weights of size markers are indicated at left.

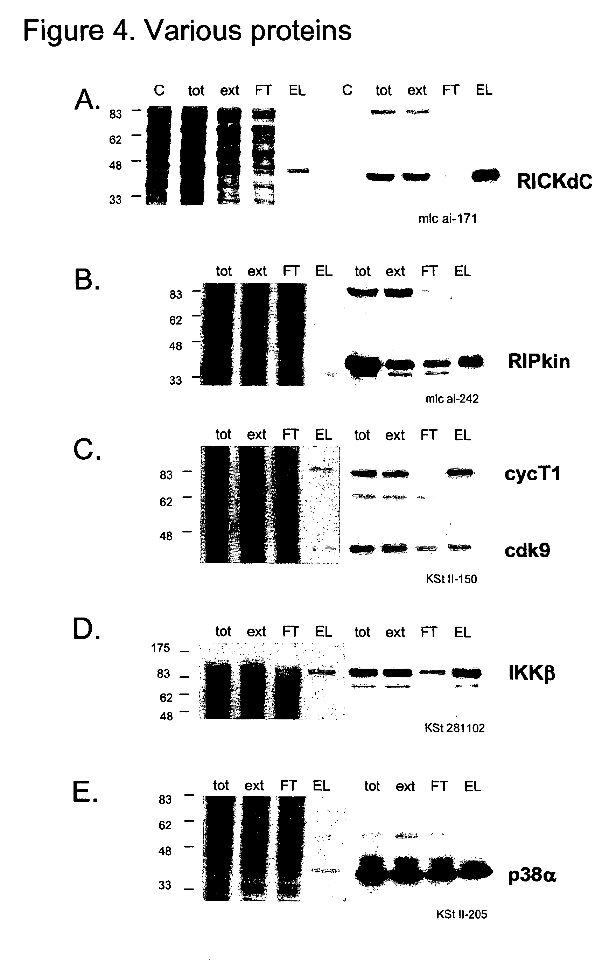

Fig. 4 A to E shows production and purification 5 mammalian kinases. Proteins were expressed in 293 cells (infection with adenovirus at 1000 particles/cell, harvest at 48 hours, lysis in lysis buffer 4 at 2 ml/250 cm² flask equivalent and subjected to streptavidin-Sepharose purification as decribed in Methods). 10 µl aliquots of the total extract (tot), soluble extract (ext), material flowing through the streptavidin-Sepharose (FT), and 40 µl of the material eluted with 2.5 mM desthiobiotin (EL) were resolved on a 10% acrylamide SDS gel, the gel was transferrred to nitrocellulose, stained with Ponceau S (total protein, left panels), blocked with NET/gelatin, probed with streptavidin-horseradish peroxidase (Amersham) and visualized by ECL (right panels). A. RICKwtdCst; B. RIP kinase domain (RIPkin); C. cdk9/cyclin T1; D. IKKβ; E. p38α.

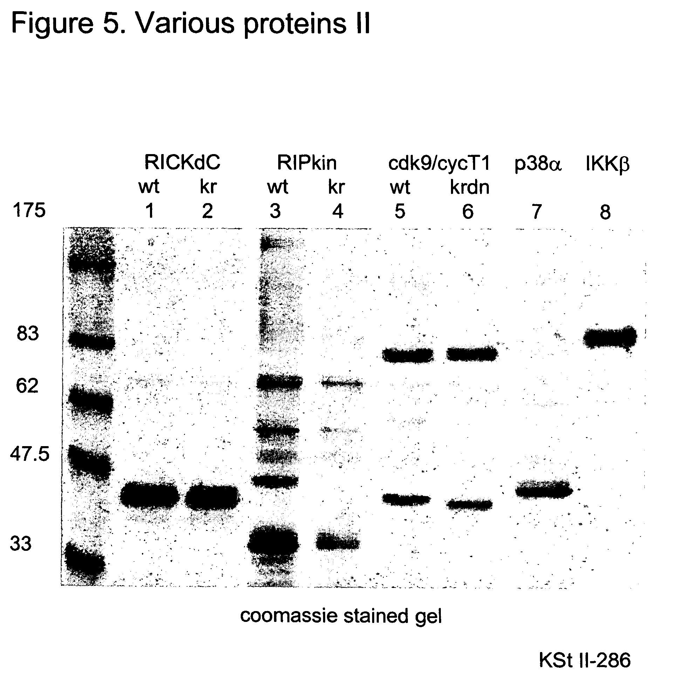

Fig. 5 shows samples of the purified forms of 5 mammalian kinases. Proteins were produced as described in Fig. 4. Aliquots of the purified material were resolved on a 10% acrylamide SDS gel and stained with colloidal Commassie Blue (Sigma). Lane 1. RICKwtdCst; Lane 2. RICKkrdCst; Lane 3. RIPwt kinase domain; Lane 4. RIPkr kinase domain; Lane 5. cdk9wt/cyclin T1; Lane 6. cdk9krdn/cyclin T1 wt; Lane 7. p38α; Lane 8. IKKβ.

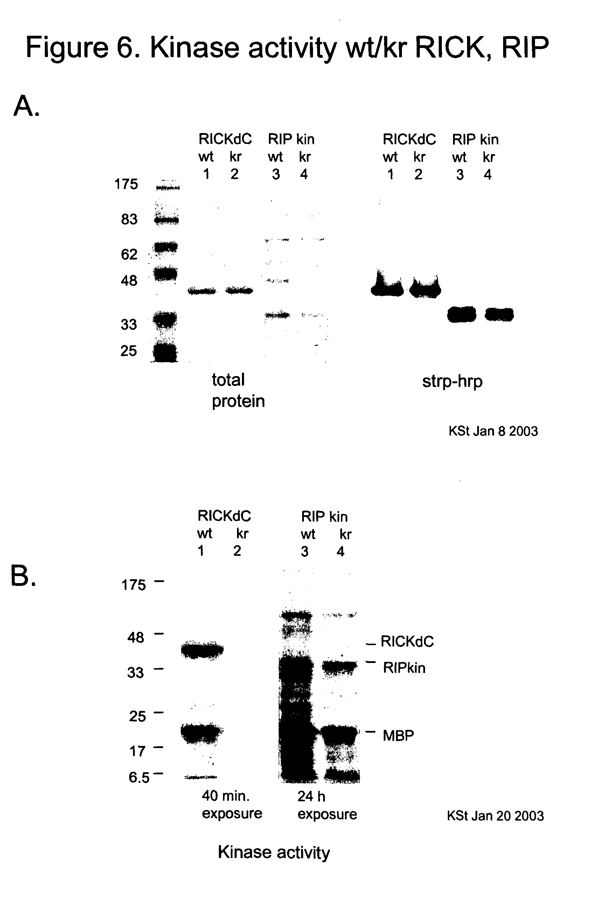

Fig. 6 shows the kinase activity of wildtype vs. kinase mutant forms of purified RICK and RIP kinase domains.

Panel A: Protein content. RICKdC and RIPkin (both wildtype and kinase inactive forms) were expressed in 293 cells, extracted and purified by streptavidin sepharose. Similar quantities of each protein were resolved by SDS-PAGE and stained for total protein (left side) or transferred to nitrocellulose, stained with streptavidin-HRP followed by ECL (right side). Panel B: Kinase activity. Equal quantities of RICKdC (wr or kr) and RIPkin (wt or kr) were incubated under RIP kinase conditions (see methods) in the presence of gamma ³³P-ATP and myelin basic protein (MBP) as a substrate. The protein reactions were resolved by SDS-PAGE, and the radioactive pattern was revealed by exposure to X-ray film for either 40 minutes (RICK samples) or 24 hours (RIP samples).

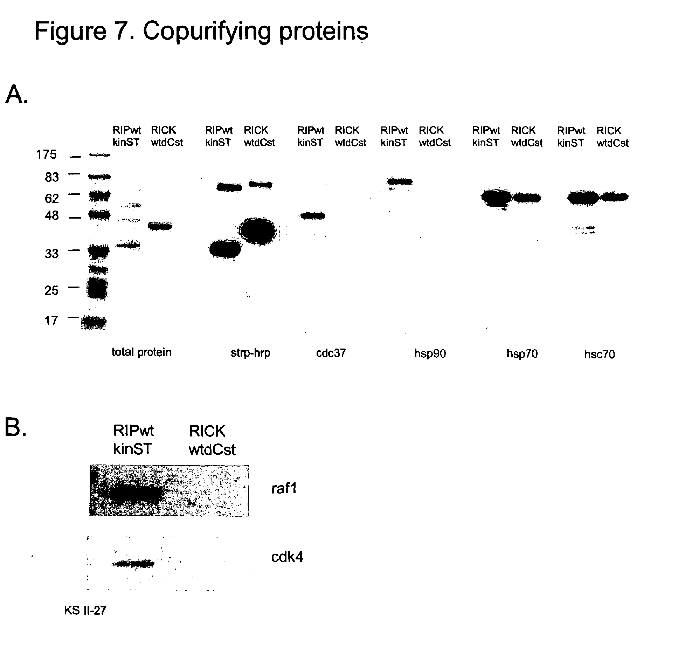

Fig. 7 shows the co-purifyed proteins found in RICK and RIP kinase preparations. RICKdC and RIPkin (both as wildtype kinase) were expressed in 293 cells, extracted and purified by streptavidin sepharose. Similar quantities of each protein were resolved by SDS-PAGE and stained for total protein (left side) or transferred to nitrocellulose, stained with either streptavidin-HRP or the indicated antisera followed HRP-conjugated secondary antibody and then by ECL.

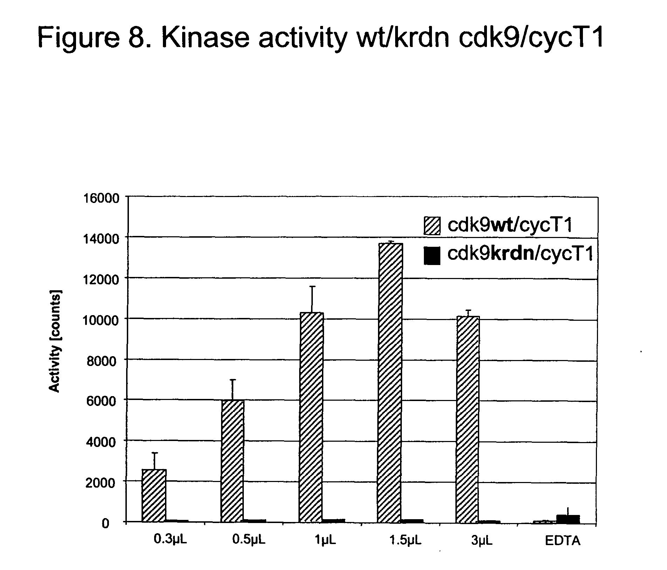

Fig. 8 shows the kinase activity of wildtype vs. kinase mutant forms of cdk9/cyclin T1 complexes. Equal quantities of cdk9/cycT1 (wt or KRDN) were incubated under cdk9 kinase conditions in the presence of gamma ³³P-ATP and an RNA polymerase II carboxy-terminal peptide as a substrate (see Methods).

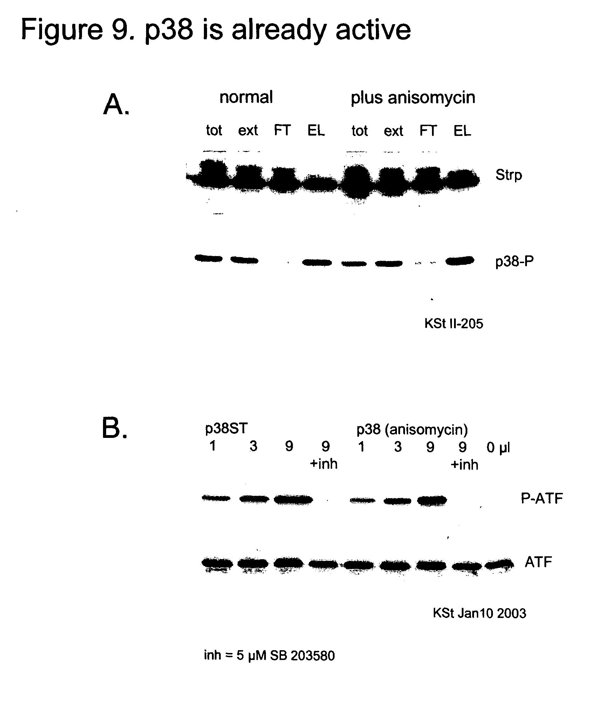

Fig. 9 shows the map kinase p38α is produced in an already active form using this system. The map kinase p38α was generated by infection with recombinant adenovirus and purifed from 293 cells as described above. In one set of cells, the MAP kinase pathway was deliberately activated by treatment of the cells with anisomycin (10 µg/ml) for 1 hour prior to harvesting the protein. Panel A (upper) shows that equal quantities of p38α were produced from both sets of cells (compare EL normal with EL plus anisomycin). Panel A (lower) shows that p38α activation, as measured by phospho-p38 staining, is similar between the two sets of samples. Panel B shows that the kinase activity of the two preparations of p38a, as measured by phosphorylation of a p38α substrate ATF, is similar between the two preparations of p38α.

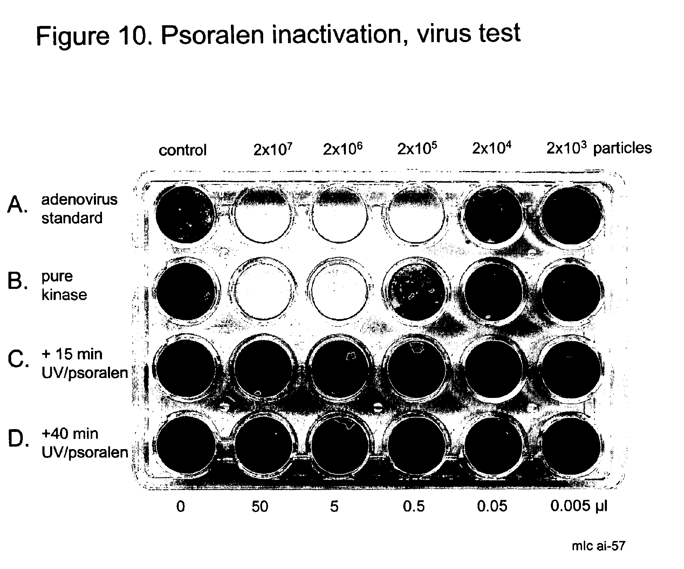

Fig. 10 shows the cytopathic effect assay of replicating adenovirus content before and after psoralen/UV treatment. 293 cells (40,000 cells/well, 24 well plate) were exposed to the indicated particles/cell MOI of pure adenovirus (row A) or 50, 5, 0.5, 0.05 and 0.005 µl of pure RICKwtdCst kinase (El1 fraction from streptavidin-Sepharose fraction, row B) or the same quantity of kinase purified from lysates exposed to 8-methoxy psoralen/265 nm UV light for 15 (row C) or 40 minutes (row D). At 7 days post-infection the cells were fixed with formaldehyde and stained with crystal violet. See Methods section for details.

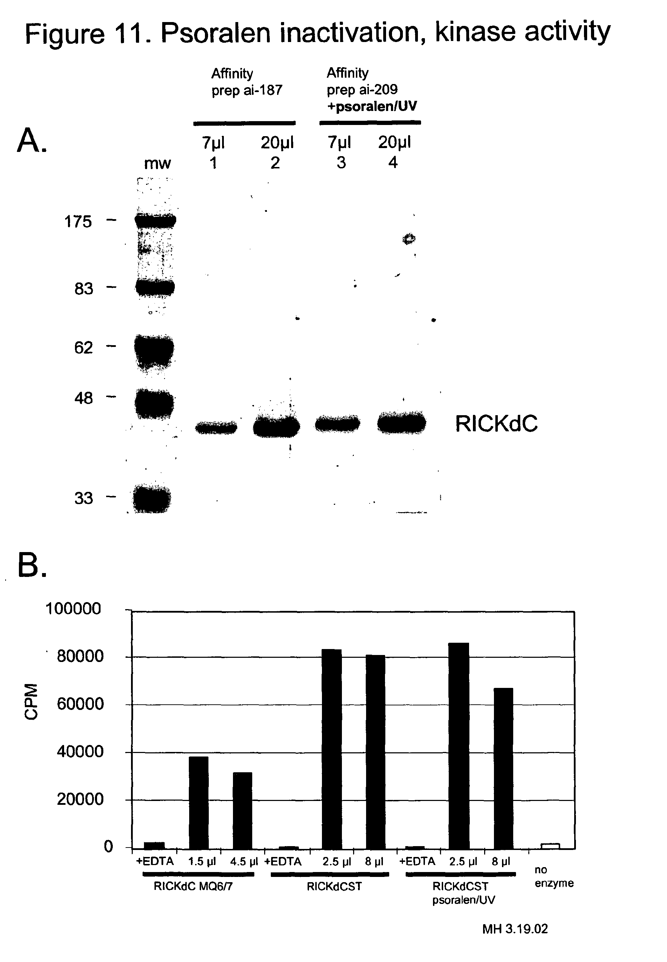

Fig. 11 shows RICK kinase activity is not impaired by psoralen/UV treatment. RICKwtdCst was purified from control lysate or lysate treated for 40 minutes with psoralen/UV. Equal protein quantities were resolved on 10% acrylamide SDS gel and stained for total protein (Coomassie blue, Panel A). Kinase reactions were performed including histone 2b as a substrate. The incorporation of radioactivity into protein in the samples was analyzed after binding the proteins to Flashplates (Panel B).

### Examples

### Materials and Methods

### Construction and purification of recombinant adenoviruses

Recombinant E1/E3 defective adenoviruses were constructed using homologous recombination in E. coli (Chartier et al., 1996, complete list of references cited at the end of the description). All viruses contained an expression cassette comprising the CMV immediate early promoter driving the open reading frame of interest followed by a rabbit ß-globin intron/polyadenylation signal, derived from the plasmid pSTC (Severne et al., 1988). The expression cassette is inserted into an adenovirus type 5 genome in place of nucleotides 342-3523, which removes the E1 region. In addition the genomes contain a deletion of adenovirus 5 E3 region of nucleotides 28592 to 30472, a destruction of the Spe I site at nucleotide 27082 and the addition of Spe I linkers at each terminal repeat. Recombinant viral genomes were released from the plasmid backbone by digestion with Spe I, purified by phenol/chloroform extraction and passage over a gel filtration column (Pharmacia Nick column). Virus cultures were initiated by transfecting the linearized genomes into 293 cells using PEl (Michou et al., 1999). After amplification of the culture, virus was purified by banding twice on CsCl gradients, transferred into HBS / 40% glycerol by passage over a gel filtration column and stored at -80°C as previously described (Cotten et al., 1996). Viral quantitation was based on protein content using the conversion 1 mg viral protein equals 3.4 x 10¹² virus particles (Lemay et al., 1980).

### Open reading frames

All open reading frames were generated by PCR from commercial cDNA preparations and cloned into derivatives of pPM7 followed by assembly into adenovirus (see above). The following cDNAs were used:

The full length wild type RICK open reading contained a cDNA identical to Genbank accession number AF027706 with a carboxy terminal haemagglutinin (HA) epitope tag. Kinase essential lysine residue 47 was mutated to arginine (K47R) to generate a kinase inactive form of the protein. RICK deleted of CARD domain (RICKdC) was generated from either the kinase wt or kinase dead (K47R) genes and contained the first 353 amino acid residues of RICK with either a carboxy terminal HA tag (RICKdC, wt or kr) or with a carboxy terminal streptag (RICKwtdCst, RICKkrdCst).

The RIP kinase domain construct (RIPkinwtST) contained a cDNA encoding residues 1-300 of the RIP protein identical to the sequence described in Genbank accession number NM_003804 plus a carboxy terminal streptag. The kinase dead version (RIPkinKRst) has the essential lysine 45 mutated to arginine (K45R) 003804 plus a carboxy terminal streptag.

The cdk9 construct contained a cDNA identical to Genbank accession number P50750, and NM_001261; a kinase dead version of the gene was prepared by mutating both kinase essental lysine 48 to arginine and the aspartic acid 167 to asparagine in cdk9KRDN. The cyclin T1 construct contained a cDNA identical to cyclin T1 (Genbank accession number XM_028819). Wildtype cdk9, KRDN cdk9 and cyclin T1 were prepared with a carboxy terminal streptag.

The p38alpha construct contained a cDNA identical to the sequence described in Genbank accession number L35253 plus a carboxy terminal streptag.

The IKKbeta construct contained a cDNA identical to Genbank accession number AF080158 and AF031416 plus a carboxy terminal streptag.

### Extraction of protein

Infected cells were harvested, washed twice with HBS and stored frozen at -20°C after flash freezing in dry ice. The frozen cell pellet was thawed on ice with 2 ml lysis buffer per 3 x 10⁷ cells (= one 185 cm² flask equivalent), pipetting gently to disperse cells. For lysis, extraction was performed on ice for 45 minute with vortexing every 5-10 minutes. The material was then sonicated in a bath sonicator for 5 minutes to fully disrupt the cells and shear the chromosomal DNA. An aliquot of the material, after full suspension, is saved as the total sample. The material was then centrifuged 10 minutes at 3300 RPM and the supernatant retained as extract.

Buffers: Lysis buffer 4: 20 mM HEPES NaOH pH 7.5, 150 mM NaCl, 20 mM 2-glycerophosphate, 10 mM NaF, 0.5% NP-40, 2 mM MgCl₂, 2 mM EGTA, 1 mM Na₃VO₄, 2 mM DTT, 1 mM PMSF, protease inhibitor cocktail (SIGMA or Roche) with the last three components added just before use. Lysis buffer 5 : 20 mM Tris-HCl pH 7.0, 20 mM 2-glycerophosphate, 10 mM NaF, 0.5% NP-40, 2 mM MgCl₂, 2 mM EGTA, 1 mM Na₃VO₄, 2 mM DTT, 1 mM PMSF, protease inhibitor cocktail (SIGMA or Roche). Lysis buffer 7: identical to Lysis buffer 4 with 400 mM NaCl. New storage buffer II (NSBII): 20 mM HEPES NaOH pH 7.5, 150 mM NaCl, 10% glycerol, 10 mM NaF, 2 mM MgCl₂, 0.1 mM EGTA, 2 mM DTT, protease inhibitor cocktail (Roche) with the last two components added just before use.

Protein production using Streptavidin-Sepharose or Streptactin macropore Streptavidin-Sepharose (Amersham Pharmacia) or Streptactin macropore (IBA) was washed with the approriate lysis buffer (at least 2 x 10 resin volumes). Washed resin was mixed with extract (100 µl of packed resin per 185 cm² flask equivalent of extract) for 60 minutes on ice with frequent gentle agitation to keep the resin dispersed. The resin plus extract were then centrifuged at 800 RPM for 3 minutes and the supernatant was saved as the flowthrough (FT). The resin was washed with 2 x 7 resin volumes of lysis buffer 4 and 2 x 7 resin volumes New storage buffer II (NSBII, supernatants saved as Wash 1 and 2) and the resin was then transferred to a disposable chromotography column (BioRad) column. The resin was then eluted twice with 7 resin volumes of NSBII+2.5 mM desthiobiotin (Eluate 1 and Eluate 2). The eluates were stored in aliquots at -80°C after flash freezing in dry ice.

### Chromatographic purification of RICKdC

Cell lysis was performed as above using lysis buffer 5. Extracts were inactivated with psoralen (see below) and then fractionated over Red Sepharose (binding in lysis buffer 5, elution with 300 mM NaCl). The salt content was diluted to 50 mM and the material was resolved on Q sepharose using a NaCl gradient from 50-500 mM. Fractions were assayed for RlCKdC content by western and for RICK autophosphorylation and histone 2b (H2b) or MBP phosphorylation.

### Psoralen inactivation

The method used conditions similar to those previously described (Cotten et al., 1994). Lysates were transferred to Nunc 24 well cell culture plates (0.75-1.5 ml per well) and 1/200 volume of 33 mg/ml 8-methoxy psoralen (in DMSO) was added per well. The psoralen precipitate which forms was distributed in the sample by pipetting and the plate was irradiated with 365 nm UV light (plate on ice, lid on) for 30 minutes.

### Analysis of virus content

Serial dilutions of test samples were performed in MEMalpha, 2% horse serum and added to 293 cells (40,000 cells/well, 24 well plate) for 2 hours at 37°C. The medium was then changed to MEMalpha, 10% newbom calf serum. At 6-8 day post infection, cells were inspected microscopically for the presence of adenovirus (plaque formation, swollen, detached cells etc) and then medium was removed, the cells were fixed for 5 minutes (1% formaldehyde, 150 mM NaCl), washed once with PBS and then stained for 10 minutes with crystal violet (3% w/v in 150 mM NaCl), washed twice with PBS, once with distilled water and scanned or photographed.

### Kinase assays

RICK and RIP, MBP and autophosphorylation and Flashplate assay. Recombinant enzyme was incubated in 40 µl containing 50 mM HEPES, pH 7.5, 1mM MgCl₂, 1mM MnCl₂, 1.5 µM ATP, 1 µCi gamma³³P-ATP/probe. For autophosphorylation analysis, the samples were then incubated with 0.1 %SDS at 99°C and resolved by SDS-PAGE (10% gel), fixed, stained and visualized by autoradiography. For Flashplate analysis, the samples included 20 ng/µl H2b. Reactions were performed in a Flashplate (basic) well for 1 hour at room temperature, the reaction was stopped by the addition of EDTA to 100 mM, washed 4 times with 100 mM EDTA, 100 mM sodium phosphate, pH 7.2, 0.01% Tween 20. Bound radioactivity was measured.

CDK9 assay. Kinase assay reactions were performed essentially as described in (Grana et al., 1994). Purified enzyme was prediluted with buffer A (20 mM HEPES, pH 7.4; 4 mM MgCl₂; 1 mM DTT) the reaction was initiated by adding 25 µl diluted enzyme to an equal volume of buffer A containing substrate (1 mM cold ATP; 1 µ Ci/well; 1 µg/well GST-CTDII [GST fused to a triple repeat of YSPTSPS]). After one hour incubation at room temperature, the assay was stopped by adding 5 µl of 0.5 M EDTA, pH 8.0, half of the reaction volume was applied to a MAIP filter plate according the manufacturers recommendations (Millipore GMBH). The dried plate was soaked with scintillation liquid, sealed with tape and incorporated radioactivity was measured employing a microbeta topcounter (Wallac Inc.).

### Westem analysis

Proteins were resolved on SDS-PAGE and transferred to nitrocellulose. A total protein image was obtained after staining with Ponceau S. Blots were then blocked overnight in NET-gelatin, and probed with the indicated antibodies, or streptavidin-horseradish peroxidase. Where appropriate, primary antibody binding was followed by secondary antibody-horseradish peroxidase. Finally, the images were developed using ECI reagents (Amersham).

### Optimization of protein expression

Most uses of recombinant adenovirus apply the virus to cells that do not support virus replication. However, for high level protein production, it may be useful to employ conditions that take advantage of both virus amplification and the inhibition of host translation that occurs during a productive adenovirus replication (Yoder et al., 1983; O'Malley et al., 1989; Huang and Schneider, 1990). Intuitively, if virus amplification occurs, one would use a low virus to cell ratio and indeed, if amplification and growth of the recombinant virus is the goal, then using more than 10-100 virus particles per cell is wasteful, because no further amplification of virus occurs at higher multiplicities of infection (MOI). However, for protein production, the parameters are indeed different.

The expression of a transgene protein was monitored as a function of various virus to cell ratios. The yield of transgene product was found to increase as one titrates from 100 to 3000 particles/cell (Fig. 1A, mlc ai-202). A comparison with the same MOls in cells that do no support virus replication (A549) demonstrated also that maximum transgene product is obtained at the highest MOl (3000 particle/cell). However, in stong constrast with 293 cells, the yield dropped dramatically and the transgene product was not abundant at less than 3000 particles/cell (Fig. 1B, mlc ai-203), most likely because of the lack of amplification of the viral genome in these cells. The phenomenon of viral shut off of host protein synthesis also becomes apparent when comparing 293 and A549 cells. Prominent biotin-containing proteins at ca. 70 kd are seen in both 293 and A549 uninfected cell extracts. The relative abundance of these proteins declines in the 293 cell extracts but not in the A549 cell extracts at higher MOls, resulting in a higher transgene/extract ratios for the 293 cell system. An analysis of the appropriate time of harvest after infection demonstrated an increase in transgene production from 24 to 48 hours, but no further increase at 72 hours (Fig. 1C, mlc ai-205). In conclusion, optimum transgene protein production is obtained using adenoviral replication conditions at relatively high MOI (1000 particles/cell) and harvest after 48 hours. Higher levels of transgene product are obtained at 10,000 particles/cell, however, the effort required to generate sufficient virus stocks becomes limiting at this MOI.

### Protein extraction, RICK

In order to purify a recombinant, overexpressed protein, it is useful to obtain the protein in a soluble, extractable form. It is often the case that expression or overexpression of a protein outside of its normal environment leads to protein misfolding, aggregation and difficulties in extraction. In addition, many complex eukaryotic proteins contain motifs important for interacting with cellular structures, and these interactions may interfere with extraction of the protein. Adenovirus expression of full-length RICK generates reasonable quantities of the protein (Fig. 2, (mlc ai-117) RICKwt total), but this form of the protein remains with the cellular debris when cells are lysed with gentle conditions (see absence of RICKwt signal in Extract). RICK contains a C-terminal CARD domain important for homodimerization or interaction with other CARD domain-containing proteins (Inohara et al., 1998; McCarthy et al., 1998; Thome et al., 1998; reviewed in Hofmann et al., 1997; Weber and Vincenz, 2001). It was found that removal of the RICK CARD domain leads to a modest increase in total protein expression (Fig. 2, RICKdC samples in total) but most importantly, this truncated form of RICK is easily extractable with non-denaturing conditions (Fig. 2, Extract).

### Purification by standard chromatography methods

The quantity of soluble recombinant protein generated with adenovirus is sufficient to support a simple two-step chromatographic purification. For this purpose, a variety of ion exchange and dye affinity resins were tested for their ability to bind RICKdC and the quaternary amine resin Mono Q Sepharose and the dye/ion exchange resin Red Sepharose were chosen for further development. A typical purification, monitoring both RICKdC kinase activity and total protein is demonstrated in Fig. 3. RICKdC present in the soluble extract possesses autophosphorylation activity (Fig. 3 extract, kinase activity). This activity and the corresponding Coomassie-staining band are bound to Red Sepharose, eluted with higher NaCl concentrations (Fig. 3, Red eluent), and subsequently are bound and eluted from Mono Q Sepharose (Fig. 3, MQ eluent). Thus, with the appropriate infection conditions, recombinant adenovirus can be used to generate sufficient quantities of recombinant protein to allow purification by standard biochemical methods.

### Affinity purification

The previous section demonstrated that recombinant adenovirus can be used to generate sufficient quantities of a recombinant enzyme to support biochemical purification of the enzyme. However, because chromatography methods take advantage of molecular differences between the desired product and the rest of the proteins in the cell, each new desired product will require the development of a novel purification strategy. To avoid this effort and to simplify purification, a number of affinity purification methods have been developed (reviewed in Constans, 2002) typically using a small protein tag attached genetically to the recombinant protein and used as a specific ligand for purification. Each method has specific disadvantages and advantages (size, charge, non-specific binding problems, elution methods, cost of resin). Some years ago, a peptide was selected for its ability to bind streptavidin in a reversible fashion, and these studies led to the development of the Strep-tag system (Schmidt and Skerra, 1994; Schmidt et al., 1996; reviewed in Skerra and Schmidt, 2000). A major advantage of this system lies in the relative affinity of the strep-tag epitope for streptavidin binding. This affinity is high enough to allow efficient purification of tagged proteins from a cellular extract, yet the affinity is still several orders of magnitude weaker than then biotin/streptavidin interaction, allowing the elution of the tagged protein from streptavidin by competition with free biotin or biotin derivatives (see Skerra and Schmidt, 2000). To test this system in the context of recombinant adenovirus, the RickdC open reading frame with either the wildtype kinase domain or the kinase inactive version (KR mutant) were fitted with a C-terminal, 9 amino acid strep-tag (Skerra and Schmidt, 2000) and cloned into an E1/E3 defective Ad5 genome. Growth and amplification of the virus was by standard methods.

It was found that similar to the non-tagged versions, the strep-tagged proteins were expressed to high levels and in a form extractable with modest conditions (Fig. 3b (mlc ai-171), compare total to extract). The material was loaded on streptavidin-Sepharose, washed extensively with the lysis buffer followed by a kinase storage buffer (KSB II). Total protein staining and staining with HRP-streptavidin both reveal that a single protein band of the expected molecular weight (41 kD) is eluted from the resin with 2.5 mM desthiobiotin (Fig. 3B, El1, EI2). The eluted fraction demonstrated RICK kinase activity as measured by autophosphosphorylation (Fig. 4B, lane 1) and a quantitative analysis of phosphorylation of H2b demonstrates that the kinase activity of the strep-tag purified material is comparable to RICK kinase activity purified by ion exchange chromatography (Figure 11B, compare RICKdC MQ purified by Red and MonoQ Sepharose to RICKdcST purified by streptavidin-Sepharose).

### Co-purifying eukaryotic kinases

For kinase production, a major justification for the use of bacterial systems, and a concern with the use of mammalian or insect systems is the issue of co-purifying cellular kinases. Because E.coli lacks classical serine/threonine and tyrosine kinases, there is little concern about co-purifying a host kinase with the target kinase. However, with mammalian kinases overexpressed in eukaryotic cells, there is the possibility that either specific or nonspecific interactions of the overexpressed kinase with cellular factors can lead to the co-purification of proteins in addition to the target kinase. This is especially important when producing kinases for drug screening efforts in which a contaminating kinase can strongly interfere with the outcome of the effort. For this reason it is important that for each kinase, a mutated version of the protein is prepared with the kinase activity disrupted, but the remainder of the protein identical to the active kinase. This can be easily done by mutating either the highly conserved lysine residue that forms part of the ATP binding pocket, or by mutating the aspartic acid residue in the kinase motif (reviewed in Hanks and Hunter, 1995). The mutant protein is purified and a measurement of residual kinase activity indicates the quantity of co-purifying cellular kinases.

This strategy was followed with RICKDC (1-353) with the essential lysine 47 mutated to arginine (K47R), with the RIP kinase domain 1-300, with the essential lysine 45 mutated to arginine (K45R) and with cdk9 with the essential lysine and aspartic acid residues mutated to arginine and asparagine (K48R, D167N). Similar quantities of RICK WT or RICK KR were purified (Figure 6a) and subjected to kinase activity analysis (Figure 6B). No detectable kinase activity was found associated with the RICK KR preparation (compare Figure 6B, lanes 1 and 2) demonstrating the purity of the RICK preparation. The cellular kinase RIP was prepared using the same methods (Figure 6A). Although the wild type kinase showed kinase activity, (Fig. 6B, lane 3) a substantial amount of kinase activity was also found associated with the kinase dead version of RIP (RIP KR Fig. 6B, lane 4).

One likely explanation for this result is the co-purification of other cellular kinase activities. Indeed, the total protein staining pattern of the purified RIP preparations revealed a number of additional protein species (Fig. 6A, lanes 3 and 4). These additional proteins were examined in more detail. The chaperone protein cdc37 interacts with a number of kinases and facilitates the appropriate folding in complexes with hsp90 such as Raf, (Silverstein et al., 1998; Grammatikakis et al., 1999), Akt (Basso et al., 2002), the IKK complex (Chen et al., 2002), cdk9/cycT1 (O'Keeffe et al., 2000), and cdk4 and cdk6 (Lamphere et al., 1997). Purified RIP contained stoichiometric quantities of 40 and 90 kD proteins, western analysis demonstrated that indeed the purified RIP (but not similarly purified RICK) contained both cdc37 and hsp90 (Fig. 7a). Because both RICK and RIP preparations contained detectable quantities of the inducible (hsp70) and consitituve (hsc70) 70 kilodalton heat shock protein, this family of chaperone could not be the basis for the RIP contamination problem. However, because cdc37/hsp90 can form multimeric complexes with a number of additional kinases, their presence was tested for in the RIP preparations. Indeed, cdk4 and raf1, two known cdc37 binding kinases (Lamphere et al., 1997; Silverstein et al., 1998; Grammatikakis et al., 1999), were present in the purified RIPwt and RIPkr preparations but not in similarly purified RICK preparations (Fig. 7B). The absence of detectable cdk2 in either RICK or RIP preparations (Fig. 7B) is consistent with the published lack of interaction between this kinase and cdc37 (Lamphere et al., 1997). Thus it is likely that the presence of the kinases cdk4 and raf1 and perhaps additional cdc37 binding kinases could account for the kinase activity observed with purified, kinase dead RIPkr. Fortunately, this phenomenon of co-purifying kinases is rather the exception than the rule. To date, all other kinases that were purified using these methods have shown negligible kinase activity associated with the kinase dead mutant form.

### The purification of enzyme complexes

It can be useful to generate and purify multi-protein complexes. For example, the cellular kinase cdk9 requires the interaction with the cyclin T1 for activity as the transcription elongation factor P-TEFb (Peng et al., 1998; Wei et al., 1998). Because maturation of cdk9 activity requires interaction of cdk9 with the cyclin T1 during synthesis and folding (O'Keeffe et al. 2000) it was chosen to co-express the two components. Indeed, mixing experiments with purified single components (cdk9 and cyclin T1) failed to generate active kinase (results not shown).

Conditions for optimum expression and extraction cdk9/cyclinT1 were identified; best yields were obtained by extracting non-frozen cells with 400 mM NaCl and harvesting the strep-tagged proteins by affinity chromatography. Because the cyclinT1 component bears the affinity tag, it was important to demonstrate that other kinases reported to interact with the cyclin (and thus would be purifed via cyclinT1) are not in the preparation. Immunoblot analysis demonstrated the absence of cdk2 and cdk4, furthermore the purified kinase activity was not inhibited by established cdk 2 and 4 (results not shown). The kinase dead mutant of cdk9 was purifed with the essential lysine and aspartic acid residues mutated to arginine and asparagine (K48R, D167N). Further supporting that the sole kinase activity in the preparation is cdk9, the cdk9 (KRDN) was expressed with cycT1, purified to the same extent as the wildtype kinase/cycT1 complex and assayed for kinase activity. No detectable kinase activity was present in the cdk9KRDN mutant protein preparation (Fig. 8) even when the sample was assayed at 10-fold higher concentration than the wildtype cdk9 (compare 0.3 µl of WT vs 3 µl of KRDN). This demonstrates that the kinase preparation is suitable for screening activities seeking cdk9 specific inhibitors.

### Contaminating adenovirus

Although the recombinant adenoviruses used here are replication-defective and pose no health threat, in many countries, recombinant adenovirus and all material derived from these viruses are required to be handled with appropriate containment. One can calculate that a typical lysate produced under the conditions described contains 10¹⁰ to 10¹² virus particles per ml and even the extensive purification allowed by affinity chromatography is not sufficient to remove virus to less than 1 in 10¹²/ml. It may be even more difficult with ion exchange chromatography where the negatively-charged adenovirion can co-purify with many negatively-charged proteins. Thus, a method of chemically inactivating the adenovirus present in the protein preparations was sought. This method must be effective at destroying a non-enveloped DNA virus, like the adenovirus, must not be hazardous to the enzymes activity that was seeked to purify and should be convenient.

It was previously demonstrated that 8-methoxy psoralen has the ability to enter the adenovirus capsid and upon irradiation with long wavelength UV, the compound forms single and double-stranded crosslinks of the adenoviral genome that block both viral transcription and replication (Cotten et al., 1992, 1994). This provides a method of inactivating adenovirus that is accepted by clinical standards (see for example Schreiber et al., 1999). The advantage of this method for recombinant protein production is that proteins have little or no absorbance in the 360 nm (long wavelength UV) range, so that the irradiation presents no hazard to protein integrity. Indeed, it has been shown that psoralen inactivation can be used to inactivate baculovirus in a recombinant protein preparation without altering the antigenic properties of the recombinant protein (Weightman and Banks, 1999). Furthermore, the presence of psoralen has no known protein damaging capacity and the compound, if included in early steps of the purification (e.g. at the lysate stage) is easily removed during purification.

Cytopathic effect assays were performed to assess the quantity of adenovirus remaining in purified kinase preparations. In this assay, surviving (non-infected) cells stain with crystal violet, the presence of replicating adenovirus results in cell rounding, death, and detachment from the substrate (referred to as cytopathic effect, CPE) and loss of crystal violet staining. Thus, clear areas in the monolayer indicate the presence of replicating adenovirus. An analysis of virus content of an ion exchange purified kinase preparation shows complete killing of the cells with 50 and 5 µl of the lysate, (Fig. 10, row B) with substantial CPE observed microscopically at 0.05 and 0.005 µl. In comparison with a pure adenovirus standard (row A) it can be calculated that the purified kinase preparation contains between 10⁴ and 10⁵ virus particles/µl. This is not surprising, the starting extract contains ca. 10⁹ viral particles/µl (results not shown). However, if the lysate used for as the starting material for kinase preparation is treated with psoralen/UV light before purification, the replicating adenovirus content is eliminated. Treatment with 15 minutes of UV largely eliminates (row C, cytopathic effect only detectable microscopically) and exposure for 40 minutes of UV light combined with 8-methoxy psoralen completely eliminates all trace of virus replication (Fig. 10, row D). More sensitive plaque assays (not shown) and RT-PCR methods (Cotten et al., 1994) have demonstrated the elimination of viral replication and transcription with this method. There are, of course, many ways to kill adenovirus. However, it is most important to demonstrate that this method does not impair the function of the recombinant protein that is sought. The enzymatic activity of a recombinant kinase thus serves as a sensitive monitor of protein integrity. As shown in Fig. 10, the 30 minute exposure to UV plus psoralen has no deleterious effect on the yield and purity of RICKdC (Fig. 11A, total protein staining) nor does it have any influence of RICK kinase activity (Fig. 11B). Similar results were obtained with a variety of other mammalian kinases including SRPK2, cdk9/cyclinT1, the cytomegalovirus kinase UL97 as well as EGFP and luciferase; in no case could an inhibition of enzyme activity (or fluorescence activity with EGFP) be detected after psoralen/UV treatment (results not shown). In conclusion, it is demonstrated that psoralen/UV treament can be used to eliminate the replicating adenovirus content of kinase preparations derived from recombinant adenovirus. The method is rapid, requiring only an addition of psoralen and a 30 minute irradiation, and functions in the presence of total cellular lysate proteins, thus, the method can be performed before purification of the desired protein, subsequent affinity purification of the protein eliminates the psoralen. Most importantly, the method does not damage protein structure and enzymatic activity is not impaired by the treatment.

### Quantification

Although the yields of purified protein will vary from protein to protein, some comment on the yields obtained with this method can be made. The preparation is conveniently performed with material derived from 16-18, 185 cm² tissue culture flasks of 293 cells (ca. 3x10⁷ cells/flask), yielding 36 ml of extract and 12 ml of material (0.04-0.2 µg/µl) eluted from streptavidin-Sepharose. Thus a typical preparation would yield 0.5-2 mg of purified RICK. Perhaps a more critical parameter is the number of enzymatic assay points provided. In our applications, a 185 cm² flask yields 0.2 ml (cdk9/cyclin T1) to 2 ml (RICK) of purified protein with 1 µl sufficient for a high throughput, radioactive assay point. Thus, 200-2000 assay points can be obtained from a 185 cm² flask.

### Conclusions

Reported here are conditions that allow the use of commonly available adenovirus vectors for the production of recombinant enzymatic activity. Although the method is more complicated than simple bacterial expression systems, the method can provide enzymatically active protein when other expression systems fail to provide sufficient quantities of active enzyme (e.g for RICK and cdk9/cyclin T1). The two advantages of the method are the use of a mammalian system for expression, and this may be important if post-translational modification and activation of the enzyme are required. Secondly, the adenovirus vectors are also extremely useful for expression studies in cell culture and in animal models, thus the same vector can be used for both protein production and for experimental purposes. An essential part of this method is the chemical inactivation of residual adenovirus in the purified protein, an important issue for the use of these proteins in other applications.

### References

Basso AD, Solit DB, Chiosis G, Giri B, Tsichlis P, Rosen N. Akt forms an intracellular complex with heat shock protein 90 (Hsp90) and Cdc37 and is destabilized by inhibitors of Hsp90 function. J Biol Chem 2002 Oct 18;277(42):39858-66
Chartier C., E. Degryse, M. Gantzer, A. Dieterle, A. Pavirani, and M. Mehtali. 1996. Efficient generation of recombinant adenovirus vectors by homologous recombination in *Escherichia coli.* J. Virol. 70:4805-4810.
Chen G, Cao P, Goeddel DV.TNF-induced recruitment and activation of the IKK complex require Cdc37 and Hsp90. Mol Cell 2002 Feb;9(2):401-10
Constans, A. Protein Purification II: Affinity Tags. The Scientist 16: 37
Cotten M, Wagner E, Zatloukal K, Phillips S, Curiel DT, Birnstiel ML. High-efficiency receptor-mediated delivery of small and large (48 kilobase gene constructs using the endosome-disruption activity of defective or chemically inactivated adenovirus particles. Proc Natl Acad Sci U S A 1992 Jul 1;89(13):6094-8
Cotten M, Saltik M, Kursa M, Wagner E, Maass G, Bimstiel ML. Psoralen treatment of adenovirus particles eliminates virus replication and transcription while maintaining the endosomolytic activity of the virus capsid. Virology 1994 Nov 15;205(1):254-61
Cotten, M., Baker A., Birnstiel M.L., Zatloukal, K., Wagner, E. (1996) Adenovirus polylysine DNA conjugates. in Current Protocols in Human Genetics, Eds. N. C. Dracopoli, J. L. Haines, B.R. Korf, D.T. Moir, C.C. Morton, C.E. Seidman, J.G. Seidman, D.R. Smith; John Wiley and Sons, Inc. New York. pp. 12.3.1-12.3.33.
Grammatikakis N, Lin JH, Grammatikakis A, Tsichlis PN, Cochran BH. p50(cdc37) acting in concert with Hsp90 is required for Raf-1 function. Mol Cell Biol 1999 Mar;19(3):1661-72
Hanks SK, Hunter T.Protein kinases 6. The eukaryotic protein kinase superfamily: kinase (catalytic) domain structure and classification.FASEB J 1995 May;9(8):576-96
Hofmann K, Bucher P, Tschopp J. The CARD domain: a new apoptotic signalling motif. Trends Biochem Sci 1997 May;22(5):155-6
Huang JT, Schneider RJ. Adenovirus inhibition of cellular protein synthesis is prevented by the drug 2-aminopurine. Proc Natl Acad Sci U S A 1990 Sep;87(18):7115-9
Inohara N, del Peso L, Koseki T, Chen S, Nunez G. RICK, a novel protein kinase containing a caspase recruitment domain, interacts with CLARP and regulates CD95-mediated apoptosis. J Biol Chem 1998 May 15;273
Karlsson S, Humphries RK, Gluzman Y, Nienhuis AW. Transfer of genes into hematopoietic cells using recombinant DNA viruses. Proc Natl Acad Sci U S A 1985 Jan;82(1):158-62
Lamphere L, Fiore F, Xu X, Brizuela L, Keezer S, Sardet C, Draetta GF, Gyuris J.Interaction between Cdc37 and Cdk4 in human cells. Oncogene 1997 Apr 24;14(16):1999-2004
Lemay P., M.L. Boudin, M. Milleville, and P. Boulanger. 1980. Human adenovirus type 2 protein IIIa. I. Purification and characterization. Virology 101:131-143.
Massie B, Dionne J, Lamarche N, Fleurent J, Langelier Y. Improved adenovirus vector provides herpes simplex virus ribonucleotide reductase R1 and R2 subunits very efficiently. Biotechnology (N Y) 1995 Jun;13(6):602-8
McCarthy JV, Ni J, Dixit VM.RIP2 is a novel NF-kappaB-activating and cell death-inducing kinase. J Biol Chem 1998 Jul 3;273
Michou, A.-I., Lehrmann, H, Saltik, M. and Cotten, M. (1999) Mutational analysis of the avian adenovirus CELO, which provides a basis for gene delivery vectors. J. Virol. 73:1399-1410.
O'Keeffe B, Fong Y, Chen D, Zhou S, Zhou Q.Requirement for a kinase-specific chaperone pathway in the production of a Cdk9/cyclin T1 heterodimer responsible for P-TEFb-mediated tat stimulation of HIV-1 transcription. J Biol Chem 2000 Jan 7;275(1):279-87
O'Malley RP, Duncan RF, Hershey JW, Mathews MB. Modification of protein synthesis initiation factors and the shut-off of host protein synthesis in adenovirus-infected cells. Virology 1989 Jan;168(1):112-8
Peng J, Zhu Y, Milton JT, Price DH. Identification of multiple cyclin subunits of human P-TEFb.Genes Dev 1998 Mar 1;12(5):755-62
Schmidt TG, Skerra A. One-step affinity purification of bacterially produced proteins by means of the "Strep tag" and immobilized recombinant core streptavidin. J Chromatogr A 1994 Aug 5;676(2):337-45
Schmidt TG, Koepke J, Frank R, Skerra A. Molecular interaction between the Strep-tag affinity peptide and its cognate target, streptavidin. J Mol Biol 1996 Feb 9;255(5):753-66
Schreiber S, Kampgen E, Wagner E, Pirkhammer D, Trcka J, Korschan H, Lindemann A, Dorffner R, Kittler H, Kasteliz F, Kupcu Z, Sinski A, Zatloukal K, Buschle M, Schmidt W, Birnstiel M, Kempe RE, Voigt T, Weber HA, Pehamberger H, Mertelsmann R, Brocker EB, Wolff K, Stingl G. Immunotherapy of metastatic malignant melanoma by a vaccine consisting of autologous interleukin 2-transfected cancer cells: outcome of a phase l study. Hum Gene Ther 1999 Apr 10;10(6):983-93
Severne Y, Wieland S, Schaffner W, Rusconi S Metal binding 'finger' structures in the glucocorticoid receptor defined by site-directed mutagenesis. EMBO J 1988 Aug;7(8):2503-8
Silverstein AM, Grammatikakis N, Cochran BH, Chinkers M, Pratt WB. p50(cdc37) binds directly to the catalytic domain of Raf as well as to a site on hsp90 that is topologically adjacent to the tetratricopeptide repeat binding site. J Biol Chem 1998 Aug 7;273(32):20090-5
Skerra A, Schmidt TG. Use of the Strep-Tag and streptavidin for detection and purification of recombinant proteins. Methods Enzymol 2000;326:271-304 Thome M, Hofmann K, Burns K, Martinon F, Bodmer JL, Mattmann C, Tschopp J.ldentification of CARDIAK, a RIP-like kinase that associates with caspase-1.Curr Biol. 1998 Jul 16;8(15):885-8.
Weber CH, Vincenz C. The death domain superfamily: a tale of two interfaces? Trends Biochem Sci 2001 Aug;26(8):475-81
Wei P, Garber ME, Fang SM, Fischer WH, Jones KA.A novel CDK9-associated C-type cyclin interacts directly with HIV-1 Tat and mediates its high-affinity, loop-specific binding to TAR RNA. Cell 1998 Feb 20;92(4):451-62
Weightman SA, Banks M. Photochemical inactivation of recombinant baculovirus. J Virol Methods 1999 Aug;81(1-2):179-82
Yoder SS, Robberson BL, Leys EJ, Hook AG, AI-Ubaidi M, Yeung CY, Kellems RE, Berget SM. Control of cellular gene expression during adenovirus infection: induction and shut-off of dihydrofolate reductase gene expression by adenovirus type 2. Mol Cell Biol 1983 May;3(5):819-28

## Claims

1. A method for recombinant production of mammalian proteins comprising the following steps:
a) providing a recombinant adenovirus as expression system;
b) infecting mammalian cells with the recombinant adenovirus to produce an active mammalian protein;
c) inactivating the adenovirus by use of an UV-sensitive intercalating agent and UV irradiation; and
d) separating and/or purifying the produced mammalian protein.

2. The method according to claim 1, wherein the infection of the mammalian cells with the recombinant adenovirus produces an active mammalian protein by performance of at least one of the host-mediated step(s) during protein production to ensure either approriate folding or post-translational modification for full activity of the recombinant protein.

3. The method according to claim 1 or 2, wherein the mammalian proteins are human proteins.

4. The method according to one of the preceding claims, wherein the mammalian proteins are selected from the group comprising kinases and phosphatases.

5. The method according to one of the preceding claims, wherein the mammalian cells are human cells.

6. The method according to one of the preceding claims, wherein the adenovirus is selected from the group comprising adenovirus type 2, adenovirus type 5, adenovirus type 3, adenovirus type 7, avian adenovirus type 1, the avian adenovirus CELO, canine adenovirus, ovine adenovirus, bovine adenovirus, parvovirus, adeno-associated virus, and chicken adeno-associated virus.

7. The method according to one of the preceding claims, further comprising step e) involving an affinity tag purification of the mammalian protein.

8. The method according to one of the preceding claims, wherein the UV-sensitive intercalating agent is selected from the group comprising psoralen, furocoumarin, hypericin, and derivatives of these compounds.

9. The method according to one of the precding claims, wherein a viral inactivation level larger than 4 log₁₀ is achieved.

10. The method according to one of the preceding claims, wherein the UV irradiation reduces the activity of the mammalian protein to not less than 75%, preferably not less than 80%, more preferably not less than 85%, even more preferably not less than 90%, and particularly not less than 95% of the total activity.

11. A mammalian protein obtainable by a method according to one of claims 1 to 10.

12. The mammalian protein according to claim 11, wherein the mammalian protein is selected from the group comprising kinases and phosphatases.

13. The mammalian protein according to claim 12, wherein the mammalian protein is RICK (RIP2, CARDIAK) cdk9/cyclinT1, p38, or IKKbeta.
